# EUROPEAN PATENT APPLICATION

(11) **EP 1 413 586 A1**
(43) Date of publication of application: **28.04.2004**
(21) Application number: 02292603.4
(22) Date of filing: 21.10.2002
(51) Int. Cl.: C07K 19/00, C12N 15/62, C12N 9/10, C12N 5/10

(54) **Regulation of the Cre recombinase using a dissociation/re-association system of said recombinase**

(71) Applicant: CENTRE NATIONAL DE LA RECHERCHE SCIENTIFIQUE (CNRS), 75794 Paris Cedex 16 (FR)
(72) Inventor: Herman, Jean-Paul, 13090 Aix en Provence (FR); Jullien, Nicolas Claude Gérard, 13120 Gardanne (FR); Sampieri, Francois, 13740 Le Rove (FR)
(74) Representative: Grosset-Fournier, Chantal Catherine

(57) **Abstract**

The present invention relates to the use of:
- a first nucleotide sequence encoding a first polypeptide sequence comprising a N-terminal fragment of the P1 bacteriophage Cre recombinase, said N-terminal fragment being linked to a first peptide,
- and of a second nucleotide sequence encoding a second polypeptide sequence comprising a C-terminal fragment of said Cre recombinase, said C-terminal fragment being linked to a second peptide,
said first and second peptides being able to bind together only in the presence of a specific third molecule,
- or of vectors containing said first and second nucleotide sequences,
- or of said first and second polypeptide sequences,
for carrying out a process for regulating the activity of the P1 bacteriophage Cre recombinase, in particular in the frame of processes of inactivation or activation of the expression of a selected gene, or for inducing chromosomal deletion, or reciprocal chromosomal translocation, or for exchanging one DNA cassette for another, or for obtaining transformed cells that can be used in the frame of gene therapy, or for obtaining transgenic animals.

SEQUENCE LISTING

<110>  CNRS

<120>  CRE RECOMBINASE

<130>  IFB 02 BJ CNR CRED

<160>  50

<170>  PatentIn version 3.1

<210>  1
<211>  1029
<212>  DNA
<213>  artificial sequence

<220>
<223>  P1 bacteriophage Cre recombinase

<220>
<221>  CDS
<222>  (1)..(1029)
<223>

<400>  1
atg tcc aac ctg ctg act gtg cac caa aac ctg cct gcc ctc cct gtg        48
Met Ser Asn Leu Leu Thr Val His Gln Asn Leu Pro Ala Leu Pro Val
1               5                   10                  15

gat gcc acc tct gat gaa gtc agg aag aac ctg atg gac atg ttc agg        96
Asp Ala Thr Ser Asp Glu Val Arg Lys Asn Leu Met Asp Met Phe Arg
            20                  25                  30

gac agg cag gcc ttc tct gaa cac acc tgg aag atg ctc ctg tct gtg       144
Asp Arg Gln Ala Phe Ser Glu His Thr Trp Lys Met Leu Leu Ser Val
        35                  40                  45

tgc aga tcc tgg gct gcc tgg tgc aag ctg aac aac agg aaa tgg ttc       192
Cys Arg Ser Trp Ala Ala Trp Cys Lys Leu Asn Asn Arg Lys Trp Phe
    50                  55                  60

cct gct gaa cct gag gat gtg agg gac tac ctc ctg tac ctg caa gcc       240
Pro Ala Glu Pro Glu Asp Val Arg Asp Tyr Leu Leu Tyr Leu Gln Ala
65                  70                  75                  80

aga ggc ctg gct gtg aag acc atc caa cag cac ctg ggc cag ctc aac       288
Arg Gly Leu Ala Val Lys Thr Ile Gln Gln His Leu Gly Gln Leu Asn
                85                  90                  95

atg ctg cac agg aga tct ggc ctg cct cgc cct tct gac tcc aat gct       336
Met Leu His Arg Arg Ser Gly Leu Pro Arg Pro Ser Asp Ser Asn Ala
            100                 105                 110

gtg tcc ctg gtg atg agg aga atc aga aag gag aat gtg gat gct ggg       384
Val Ser Leu Val Met Arg Arg Ile Arg Lys Glu Asn Val Asp Ala Gly
        115                 120                 125

gag aga gcc aag cag gcc ctg gcc ttt gaa cgc act gac ttt gac caa       432
Glu Arg Ala Lys Gln Ala Leu Ala Phe Glu Arg Thr Asp Phe Asp Gln
    130                 135                 140

gtc aga tcc ctg atg gag aac tct gac aga tgc cag gac atc agg aac 480
Val Arg Ser Leu Met Glu Asn Ser Asp Arg Cys Gln Asp Ile Arg Asn
145                 150                 155                 160

ctg gcc ttc ctg ggc att gcc tac aac acc ctg ctg cgc att gcc gaa 528
Leu Ala Phe Leu Gly Ile Ala Tyr Asn Thr Leu Leu Arg Ile Ala Glu
                165                 170                 175

att gcc aga atc aga gtg aag gac atc tcc cgc acc gat ggt ggg aga 576
Ile Ala Arg Ile Arg Val Lys Asp Ile Ser Arg Thr Asp Gly Gly Arg
            180                 185                 190

atg ctg atc cac att ggc agg acc aag acc ctg gtg tcc aca gct ggt 624
Met Leu Ile His Ile Gly Arg Thr Lys Thr Leu Val Ser Thr Ala Gly
        195                 200                 205

gtg gag aag gcc ctg tcc ctg ggg gtt acc aag ctg gtg gag aga tgg 672
Val Glu Lys Ala Leu Ser Leu Gly Val Thr Lys Leu Val Glu Arg Trp
    210                 215                 220

atc tct gtg tct ggt gtg gct gat gac ccc aac aac tac ctg ttc tgc 720
Ile Ser Val Ser Gly Val Ala Asp Asp Pro Asn Asn Tyr Leu Phe Cys
225                 230                 235                 240

cgg gtc aga aag aat ggt gtg gct gcc cct tct gcc acc tcc caa ctg 768
Arg Val Arg Lys Asn Gly Val Ala Ala Pro Ser Ala Thr Ser Gln Leu
                245                 250                 255

tcc acc cgg gcc ctg gaa ggg atc ttt gag gcc acc cac cgc ctg atc 816
Ser Thr Arg Ala Leu Glu Gly Ile Phe Glu Ala Thr His Arg Leu Ile
            260                 265                 270

tat ggt gcc aag gat gac tct ggg cag aga tac ctg gcc tgg tct ggc 864
Tyr Gly Ala Lys Asp Asp Ser Gly Gln Arg Tyr Leu Ala Trp Ser Gly
        275                 280                 285

cac tct gcc aga gtg ggt gct gcc agg gac atg gcc agg gct ggt gtg 912
His Ser Ala Arg Val Gly Ala Ala Arg Asp Met Ala Arg Ala Gly Val
    290                 295                 300

tcc atc cct gaa atc atg cag gct ggt ggc tgg acc aat gtg aac att 960
Ser Ile Pro Glu Ile Met Gln Ala Gly Gly Trp Thr Asn Val Asn Ile
305                 310                 315                 320

gtg atg aac tac atc aga aac ctg gac tct gag act ggg gcc atg gtg 1008
Val Met Asn Tyr Ile Arg Asn Leu Asp Ser Glu Thr Gly Ala Met Val
                325                 330                 335

agg ctg ctc gag gat ggg gac 1029
Arg Leu Leu Glu Asp Gly Asp
            340

<210> 2
<211> 343
<212> PRT
<213> artificial sequence

<220>
<223> P1 bacteriophage Cre recombinase

<400> 2
Met Ser Asn Leu Leu Thr Val His Gln Asn Leu Pro Ala Leu Pro Val
1               5                   10                  15

Asp Ala Thr Ser Asp Glu Val Arg Lys Asn Leu Met Asp Met Phe Arg
            20                  25                  30

Asp Arg Gln Ala Phe Ser Glu His Thr Trp Lys Met Leu Leu Ser Val
        35                  40                  45

Cys Arg Ser Trp Ala Ala Trp Cys Lys Leu Asn Asn Arg Lys Trp Phe
    50                  55                  60

Pro Ala Glu Pro Glu Asp Val Arg Asp Tyr Leu Leu Tyr Leu Gln Ala
65                  70                  75                  80

Arg Gly Leu Ala Val Lys Thr Ile Gln Gln His Leu Gly Gln Leu Asn
                85                  90                  95

Met Leu His Arg Arg Ser Gly Leu Pro Arg Pro Ser Asp Ser Asn Ala
            100                 105                 110

Val Ser Leu Val Met Arg Arg Ile Arg Lys Glu Asn Val Asp Ala Gly
        115                 120                 125

Glu Arg Ala Lys Gln Ala Leu Ala Phe Glu Arg Thr Asp Phe Asp Gln
    130                 135                 140

Val Arg Ser Leu Met Glu Asn Ser Asp Arg Cys Gln Asp Ile Arg Asn
145                 150                 155                 160

Leu Ala Phe Leu Gly Ile Ala Tyr Asn Thr Leu Leu Arg Ile Ala Glu
                165                 170                 175

Ile Ala Arg Ile Arg Val Lys Asp Ile Ser Arg Thr Asp Gly Gly Arg
            180                 185                 190

Met Leu Ile His Ile Gly Arg Thr Lys Thr Leu Val Ser Thr Ala Gly
        195                 200                 205

Val Glu Lys Ala Leu Ser Leu Gly Val Thr Lys Leu Val Glu Arg Trp
    210                 215                 220

Ile Ser Val Ser Gly Val Ala Asp Asp Pro Asn Asn Tyr Leu Phe Cys
225                 230                 235                 240

Arg Val Arg Lys Asn Gly Val Ala Ala Pro Ser Ala Thr Ser Gln Leu
                245                 250                 255

Ser Thr Arg Ala Leu Glu Gly Ile Phe Glu Ala Thr His Arg Leu Ile
            260                 265                 270

Tyr Gly Ala Lys Asp Asp Ser Gly Gln Arg Tyr Leu Ala Trp Ser Gly
        275                 280                 285

His Ser Ala Arg Val Gly Ala Ala Arg Asp Met Ala Arg Ala Gly Val
    290                 295                 300

Ser Ile Pro Glu Ile Met Gln Ala Gly Gly Trp Thr Asn Val Asn Ile
305                 310                 315                 320

Val Met Asn Tyr Ile Arg Asn Leu Asp Ser Glu Thr Gly Ala Met Val
                325                 330                 335

Arg Leu Leu Glu Asp Gly Asp
            340

<210>  3
<211>  258
<212>  DNA
<213>  artificial sequence

<220>
<223>  fragment delimited by the aminoacids located in
       positions 19 to 104 of the Cre recombinase

<220>
<221>  CDS
<222>  (1)..(258)
<223>

<400>  3
acc tct gat gaa gtc agg aag aac ctg atg gac atg ttc agg gac agg       48
Thr Ser Asp Glu Val Arg Lys Asn Leu Met Asp Met Phe Arg Asp Arg
1               5                   10                  15

cag gcc ttc tct gaa cac acc tgg aag atg ctc ctg tct gtg tgc aga       96
Gln Ala Phe Ser Glu His Thr Trp Lys Met Lèu Leu Ser Val Cys Arg
            20                  25                  30

tcc tgg gct gcc tgg tgc aag ctg aac aac agg aaa tgg ttc cct gct      144
Ser Trp Ala Ala Trp Cys Lys Leu Asn Asn Arg Lys Trp Phe Pro Ala
        35                  40                  45

gaa cct gag gat gtg agg gac tac ctc ctg tac ctg caa gcc aga ggc      192
Glu Pro Glu Asp Val Arg Asp Tyr Leu Leu Tyr Leu Gln Ala Arg Gly
    50                  55                  60

ctg gct gtg aag acc atc caa cag cac ctg ggc cag ctc aac atg ctg      240
Leu Ala Val Lys Thr Ile Gln Gln His Leu Gly Gln Leu Asn Met Leu
65                  70                  75                  80

cac agg aga tct ggc ctg                                              258
His Arg Arg Ser Gly Leu
                85

<210>  4
<211>  86
<212>  PRT
<213>  artificial sequence

<220>
<223>  fragment delimited by the aminoacids located in
       positions 19 to 104 of the Cre recombinase

<400>  4
Thr Ser Asp Glu Val Arg Lys Asn Leu Met Asp Met Phe Arg Asp Arg
1               5                   10                  15

Gln Ala Phe Ser Glu His Thr Trp Lys Met Leu Leu Ser Val Cys Arg
            20                  25                  30

Ser Trp Ala Ala Trp Cys Lys Leu Asn Asn Arg Lys Trp Phe Pro Ala
        35                  40                  45

Glu Pro Glu Asp Val Arg Asp Tyr Leu Leu Tyr Leu Gln Ala Arg Gly
    50                  55                  60

Leu Ala Val Lys Thr Ile Gln Gln His Leu Gly Gln Leu Asn Met Leu
65                  70                  75                  80

His Arg Arg Ser Gly Leu
                85


<210>  5
<211>  123
<212>  DNA
<213>  artificial sequence

<220>
<223>  fragment delimited by the aminoacids located in
       positions 19 to 59 of the Cre recombinase

<220>
<221>  CDS
<222>  (1)..(123)
<223>

<400>  5
acc tct gat gaa gtc agg aag aac ctg atg gac atg ttc agg gac agg       48
Thr Ser Asp Glu Val Arg Lys Asn Leu Met Asp Met Phe Arg Asp Arg
1               5                   10                  15

cag gcc ttc tct gaa cac acc tgg aag atg ctc ctg tct gtg tgc aga       96
Gln Ala Phe Ser Glu His Thr Trp Lys Met Leu Leu Ser Val Cys Arg
            20                  25                  30

tcc tgg gct gcc tgg tgc aag ctg aac                                   123
Ser Trp Ala Ala Trp Cys Lys Leu Asn
        35                  40


<210>  6
<211>  41
<212>  PRT
<213>  artificial sequence

<220>
<223>  fragment delimited by the aminoacids located in
       positions 19 to 59 of the Cre recombinase

<400>  6
Thr Ser Asp Glu Val Arg Lys Asn Leu Met Asp Met Phe Arg Asp Arg
1               5                   10                  15

Gln Ala Phe Ser Glu His Thr Trp Lys Met Leu Leu Ser Val Cys Arg
            20                  25                  30

Ser Trp Ala Ala Trp Cys Lys Leu Asn
        35                  40

<210>  7
<211>  714
<212>  DNA
<213>  artificial sequence

<220>
<223>  fragment delimited by the aminoacids located in
       positions 106 to 343 of the Cre recombinase

<220>
<221>  CDS
<222>  (1)..(714)
<223>

<400>  7
cgc cct tct gac tcc aat gct gtg tcc ctg gtg atg agg aga atc aga        48
Arg Pro Ser Asp Ser Asn Ala Val Ser Leu Val Met Arg Arg Ile Arg
1               5                   10                  15

aag gag aat gtg gat gct ggg gag aga gcc aag cag gcc ctg gcc ttt        96
Lys Glu Asn Val Asp Ala Gly Glu Arg Ala Lys Gln Ala Leu Ala Phe
            20                  25                  30

gaa cgc act gac ttt gac caa gtc aga tcc ctg atg gag aac tct gac       144
Glu Arg Thr Asp Phe Asp Gln Val Arg Ser Leu Met Glu Asn Ser Asp
        35                  40                  45

aga tgc cag gac atc agg aac ctg gcc ttc ctg ggc att gcc tac aac       192
Arg Cys Gln Asp Ile Arg Asn Leu Ala Phe Leu Gly Ile Ala Tyr Asn
    50                  55                  60

acc ctg ctg cgc att gcc gaa att gcc aga atc aga gtg aag gac atc       240
Thr Leu Leu Arg Ile Ala Glu Ile Ala Arg Ile Arg Val Lys Asp Ile
65                  70                  75                  80

tcc cgc acc gat ggt ggg aga atg ctg atc cac att ggc agg acc aag       288
Ser Arg Thr Asp Gly Gly Arg Met Leu Ile His Ile Gly Arg Thr Lys
                85                  90                  95

acc ctg gtg tcc aca gct ggt gtg gag aag gcc ctg tcc ctg ggg gtt       336
Thr Leu Val Ser Thr Ala Gly Val Glu Lys Ala Leu Ser Leu Gly Val
            100                 105                 110

acc aag ctg gtg gag aga tgg atc tct gtg tct ggt gtg gct gat gac       384
Thr Lys Leu Val Glu Arg Trp Ile Ser Val Ser Gly Val Ala Asp Asp
        115                 120                 125

ccc aac aac tac ctg ttc tgc cgg gtc aga aag aat ggt gtg gct gcc       432
Pro Asn Asn Tyr Leu Phe Cys Arg Val Arg Lys Asn Gly Val Ala Ala
    130                 135                 140

cct tct gcc acc tcc caa ctg tcc acc cgg gcc ctg gaa ggg atc ttt       480
Pro Ser Ala Thr Ser Gln Leu Ser Thr Arg Ala Leu Glu Gly Ile Phe
145                 150                 155                 160

gag gcc acc cac cgc ctg atc tat ggt gcc aag gat gac tct ggg cag      528
Glu Ala Thr His Arg Leu Ile Tyr Gly Ala Lys Asp Asp Ser Gly Gln
                165                 170                 175

aga tac ctg gcc tgg tct ggc cac tct gcc aga gtg ggt gct gcc agg      576
Arg Tyr Leu Ala Trp Ser Gly His Ser Ala Arg Val Gly Ala Ala Arg
            180                 185                 190

gac atg gcc agg gct ggt gtg tcc atc cct gaa atc atg cag gct ggt      624
Asp Met Ala Arg Ala Gly Val Ser Ile Pro Glu Ile Met Gln Ala Gly
        195                 200                 205

ggc tgg acc aat gtg aac att gtg atg aac tac atc aga aac ctg gac      672
Gly Trp Thr Asn Val Asn Ile Val Met Asn Tyr Ile Arg Asn Leu Asp
    210                 215                 220

tct gag act ggg gcc atg gtg agg ctg ctc gag gat ggg gac              714
Ser Glu Thr Gly Ala Met Val Arg Leu Leu Glu Asp Gly Asp
225                 230                 235


<210>  8
<211>  238
<212>  PRT
<213>  artificial sequence

<220>
<223>  fragment delimited by the aminoacids located in
       positions 106 to 343 of the Cre recombinase

<400>  8
Arg Pro Ser Asp Ser Asn Ala Val Ser Leu Val Met Arg Arg Ile Arg
1               5                   10                  15

Lys Glu Asn Val Asp Ala Gly Glu Arg Ala Lys Gln Ala Leu Ala Phe
            20                  25                  30

Glu Arg Thr Asp Phe Asp Gln Val Arg Ser Leu Met Glu Asn Ser Asp
        35                  40                  45

Arg Cys Gln Asp Ile Arg Asn Leu Ala Phe Leu Gly Ile Ala Tyr Asn
    50                  55                  60

Thr Leu Leu Arg Ile Ala Glu Ile Ala Arg Ile Arg Val Lys Asp Ile
65                  70                  75                  80

Ser Arg Thr Asp Gly Gly Arg Met Leu Ile His Ile Gly Arg Thr Lys
                85                  90                  95

Thr Leu Val Ser Thr Ala Gly Val Glu Lys Ala Leu Ser Leu Gly Val
            100                 105                 110

Thr Lys Leu Val Glu Arg Trp Ile Ser Val Ser Gly Val Ala Asp Asp
        115                 120                 125

Pro Asn Asn Tyr Leu Phe Cys Arg Val Arg Lys Asn Gly Val Ala Ala
    130                 135                 140

Pro Ser Ala Thr Ser Gln Leu Ser Thr Arg Ala Leu Glu Gly Ile Phe
145                 150                 155                 160

Glu Ala Thr His Arg Leu Ile Tyr Gly Ala Lys Asp Asp Ser Gly Gln
                165                 170                 175

Arg Tyr Leu Ala Trp Ser Gly His Ser Ala Arg Val Gly Ala Ala Arg
            180                 185                 190

Asp Met Ala Arg Ala Gly Val Ser Ile Pro Glu Ile Met Gln Ala Gly
        195                 200                 205

Gly Trp Thr Asn Val Asn Ile Val Met Asn Tyr Ile Arg Asn Leu Asp
    210                 215                 220

Ser Glu Thr Gly Ala Met Val Arg Leu Leu Glu Asp Gly Asp
225                 230                 235


<210>  9
<211>  852
<212>  DNA
<213>  artificial sequence

<220>
<223>  fragment delimited by the aminoacids located in
       positions 60 to 343 of the Cre recombinase

<220>
<221>  CDS
<222>  (1)..(852)
<223>

<400>  9
aac agg aaa tgg ttc cct gct gaa cct gag gat gtg agg gac tac ctc      48
Asn Arg Lys Trp Phe Pro Ala Glu Pro Glu Asp Val Arg Asp Tyr Leu
1               5                   10                  15

ctg tac ctg caa gcc aga ggc ctg gct gtg aag acc atc caa cag cac      96
Leu Tyr Leu Gln Ala Arg Gly Leu Ala Val Lys Thr Ile Gln Gln His
            20                  25                  30

ctg ggc cag ctc aac atg ctg cac agg aga tct ggc ctg cct cgc cct      144
Leu Gly Gln Leu Asn Met Leu His Arg Arg Ser Gly Leu Pro Arg Pro
        35                  40                  45

tct gac tcc aat gct gtg tcc ctg gtg atg agg aga atc aga aag gag      192
Ser Asp Ser Asn Ala Val Ser Leu Val Met Arg Arg Ile Arg Lys Glu
    50                  55                  60

aat gtg gat gct ggg gag aga gcc aag cag gcc ctg gcc ttt gaa cgc      240
Asn Val Asp Ala Gly Glu Arg Ala Lys Gln Ala Leu Ala Phe Glu Arg
65                  70                  75                  80

act gac ttt gac caa gtc aga tcc ctg atg gag aac tct gac aga tgc      288
Thr Asp Phe Asp Gln Val Arg Ser Leu Met Glu Asn Ser Asp Arg Cys
                85                  90                  95

cag gac atc agg aac ctg gcc ttc ctg ggc att gcc tac aac acc ctg      336
Gln Asp Ile Arg Asn Leu Ala Phe Leu Gly Ile Ala Tyr Asn Thr Leu
            100                 105                 110

ctg cgc att gcc gaa att gcc aga atc aga gtg aag gac atc tcc cgc 384
Leu Arg Ile Ala Glu Ile Ala Arg Ile Arg Val Lys Asp Ile Ser Arg
        115                 120                 125

acc gat ggt ggg aga atg ctg atc cac att ggc agg acc aag acc ctg 432
Thr Asp Gly Gly Arg Met Leu Ile His Ile Gly Arg Thr Lys Thr Leu
    130                 135                 140

gtg tcc aca gct ggt gtg gag aag gcc ctg tcc ctg ggg gtt acc aag 480
Val Ser Thr Ala Gly Val Glu Lys Ala Leu Ser Leu Gly Val Thr Lys
145                 150                 155                 160

ctg gtg gag aga tgg atc tct gtg tct ggt gtg gct gat gac ccc aac 528
Leu Val Glu Arg Trp Ile Ser Val Ser Gly Val Ala Asp Asp Pro Asn
                165                 170                 175

aac tac ctg ttc tgc cgg gtc aga aag aat ggt gtg gct gcc cct tct 576
Asn Tyr Leu Phe Cys Arg Val Arg Lys Asn Gly Val Ala Ala Pro Ser
            180                 185                 190

gcc acc tcc caa ctg tcc acc cgg gcc ctg gaa ggg atc ttt gag gcc 624
Ala Thr Ser Gln Leu Ser Thr Arg Ala Leu Glu Gly Ile Phe Glu Ala
        195                 200                 205

acc cac cgc ctg atc tat ggt gcc aag gat gac tct ggg cag aga tac 672
Thr His Arg Leu Ile Tyr Gly Ala Lys Asp Asp Ser Gly Gln Arg Tyr
    210                 215                 220

ctg gcc tgg tct ggc cac tct gcc aga gtg ggt gct gcc agg gac atg 720
Leu Ala Trp Ser Gly His Ser Ala Arg Val Gly Ala Ala Arg Asp Met
225                 230                 235                 240

gcc agg gct ggt gtg tcc atc cct gaa atc atg cag gct ggt ggc tgg 768
Ala Arg Ala Gly Val Ser Ile Pro Glu Ile Met Gln Ala Gly Gly Trp
                245                 250                 255

acc aat gtg aac att gtg atg aac tac atc aga aac ctg gac tct gag 816
Thr Asn Val Asn Ile Val Met Asn Tyr Ile Arg Asn Leu Asp Ser Glu
            260                 265                 270

act ggg gcc atg gtg agg ctg ctc gag gat ggg gac 852
Thr Gly Ala Met Val Arg Leu Leu Glu Asp Gly Asp
        275                 280

<210> 10
<211> 284
<212> PRT
<213> artificial sequence

<220>
<223> fragment delimited by the aminoacids located in
positions 60 to 343 of the Cre recombinase

<400> 10
Asn Arg Lys Trp Phe Pro Ala Glu Pro Glu Asp Val Arg Asp Tyr Leu
1               5                   10                  15

Leu Tyr Leu Gln Ala Arg Gly Leu Ala Val Lys Thr Ile Gln Gln His
            20                  25                  30

Leu Gly Gln Leu Asn Met Leu His Arg Arg Ser Gly Leu Pro Arg Pro
        35                  40                  45

Ser Asp Ser Asn Ala Val Ser Leu Val Met Arg Arg Ile Arg Lys Glu
    50                  55                  60

Asn Val Asp Ala Gly Glu Arg Ala Lys Gln Ala Leu Ala Phe Glu Arg
65                  70                  75                  80

Thr Asp Phe Asp Gln Val Arg Ser Leu Met Glu Asn Ser Asp Arg Cys
                85                  90                  95

Gln Asp Ile Arg Asn Leu Ala Phe Leu Gly Ile Ala Tyr Asn Thr Leu
            100                 105                 110

Leu Arg Ile Ala Glu Ile Ala Arg Ile Arg Val Lys Asp Ile Ser Arg
        115                 120                 125

Thr Asp Gly Gly Arg Met Leu Ile His Ile Gly Arg Thr Lys Thr Leu
    130                 135                 140

Val Ser Thr Ala Gly Val Glu Lys Ala Leu Ser Leu Gly Val Thr Lys
145                 150                 155                 160

Leu Val Glu Arg Trp Ile Ser Val Ser Gly Val Ala Asp Asp Pro Asn
                165                 170                 175

Asn Tyr Leu Phe Cys Arg Val Arg Lys Asn Gly Val Ala Ala Pro Ser
            180                 185                 190

Ala Thr Ser Gln Leu Ser Thr Arg Ala Leu Glu Gly Ile Phe Glu Ala
        195                 200                 205

Thr His Arg Leu Ile Tyr Gly Ala Lys Asp Asp Ser Gly Gln Arg Tyr
    210                 215                 220

Leu Ala Trp Ser Gly His Ser Ala Arg Val Gly Ala Ala Arg Asp Met
225                 230                 235                 240

Ala Arg Ala Gly Val Ser Ile Pro Glu Ile Met Gln Ala Gly Gly Trp
                245                 250                 255

Thr Asn Val Asn Ile Val Met Asn Tyr Ile Arg Asn Leu Asp Ser Glu
            260                 265                 270

Thr Gly Ala Met Val Arg Leu Leu Glu Asp Gly Asp
        275                 280


<210>  11
<211>  321
<212>  DNA
<213>  homo sapiens

<220>
<221>  CDS
<222>  (1)..(321)
<223>  FKBP human protein

<400> 11
gga gtg cag gtg gaa acc atc tcc cca gga gac ggg cgc acc ttc ccc 48
Gly Val Gln Val Glu Thr Ile Ser Pro Gly Asp Gly Arg Thr Phe Pro
1               5                   10                  15

aag cgc ggc cag acc tgc gtg gtg cac tac acc ggg atg ctt gaa gat 96
Lys Arg Gly Gln Thr Cys Val Val His Tyr Thr Gly Met Leu Glu Asp
            20                  25                  30

gga aag aaa ttt gat tcc tcc cgg gac aga aac aag ccc ttt aag ttt 144
Gly Lys Lys Phe Asp Ser Ser Arg Asp Arg Asn Lys Pro Phe Lys Phe
        35                  40                  45

atg cta ggc aag cag gag gtg atc cga ggc tgg gaa gaa ggg gtt gcc 192
Met Leu Gly Lys Gln Glu Val Ile Arg Gly Trp Glu Glu Gly Val Ala
    50                  55                  60

cag atg agt gtg ggt cag aga gcc aaa ctg act ata tct cca gat tat 240
Gln Met Ser Val Gly Gln Arg Ala Lys Leu Thr Ile Ser Pro Asp Tyr
65                  70                  75                  80

gcc tat ggt gcc act ggg cac cca ggc atc atc cca cca cat gcc act 288
Ala Tyr Gly Ala Thr Gly His Pro Gly Ile Ile Pro Pro His Ala Thr
                85                  90                  95

ctc gtc ttc gat gtg gag ctt cta aaa ctg gaa 321
Leu Val Phe Asp Val Glu Leu Leu Lys Leu Glu
            100                 105


<210> 12
<211> 107
<212> PRT
<213> homo sapiens

<400> 12
Gly Val Gln Val Glu Thr Ile Ser Pro Gly Asp Gly Arg Thr Phe Pro
1               5                   10                  15

Lys Arg Gly Gln Thr Cys Val Val His Tyr Thr Gly Met Leu Glu Asp
            20                  25                  30

Gly Lys Lys Phe Asp Ser Ser Arg Asp Arg Asn Lys Pro Phe Lys Phe
        35                  40                  45

Met Leu Gly Lys Gln Glu Val Ile Arg Gly Trp Glu Glu Gly Val Ala
    50                  55                  60

Gln Met Ser Val Gly Gln Arg Ala Lys Leu Thr Ile Ser Pro Asp Tyr
65                  70                  75                  80

Ala Tyr Gly Ala Thr Gly His Pro Gly Ile Ile Pro Pro His Ala Thr
                85                  90                  95

Leu Val Phe Asp Val Glu Leu Leu Lys Leu Glu
            100                 105


<210> 13
<211> 276
<212> DNA

<213>  artificial sequence

<220>
<223>  FRB protein

<220>
<221>  CDS
<222>  (1)..(276)
<223>

<400>  13
atc ctc tgg cat gag atg tgg cat gaa ggc ctg gaa gag gca tct cgt        48
Ile Leu Trp His Glu Met Trp His Glu Gly Leu Glu Glu Ala Ser Arg
1               5                   10                  15

ttg tac ttt ggg gaa agg aac gtg aaa ggc atg ttt gag gtg ctg gag        96
Leu Tyr Phe Gly Glu Arg Asn Val Lys Gly Met Phe Glu Val Leu Glu
            20                  25                  30

ccc ttg cat gct atg atg gaa cgg ggc ccc cag act ctg aag gaa aca       144
Pro Leu His Ala Met Met Glu Arg Gly Pro Gln Thr Leu Lys Glu Thr
        35                  40                  45

tcc ttt aat cag gcc tat ggt cga gat tta atg gag gcc caa gag tgg       192
Ser Phe Asn Gln Ala Tyr Gly Arg Asp Leu Met Glu Ala Gln Glu Trp
    50                  55                  60

tgc agg aag tac atg aaa tca ggg aat gtc aag gac ctc ctc caa gcc       240
Cys Arg Lys Tyr Met Lys Ser Gly Asn Val Lys Asp Leu Leu Gln Ala
65                  70                  75                  80

tgg gac ctc tat tat cat gtg ttc cga cga atc tca                       276
Trp Asp Leu Tyr Tyr His Val Phe Arg Arg Ile Ser
                85                  90


<210>  14
<211>  92
<212>  PRT
<213>  artificial sequence

<220>
<223>  FRB protein

<400>  14
Ile Leu Trp His Glu Met Trp His Glu Gly Leu Glu Glu Ala Ser Arg
1               5                   10                  15

Leu Tyr Phe Gly Glu Arg Asn Val Lys Gly Met Phe Glu Val Leu Glu
            20                  25                  30

Pro Leu His Ala Met Met Glu Arg Gly Pro Gln Thr Leu Lys Glu Thr
        35                  40                  45

Ser Phe Asn Gln Ala Tyr Gly Arg Asp Leu Met Glu Ala Gln Glu Trp
    50                  55                  60

Cys Arg Lys Tyr Met Lys Ser Gly Asn Val Lys Asp Leu Leu Gln Ala
65                  70                  75                  80

Trp Asp Leu Tyr Tyr His Val Phe Arg Arg Ile Ser
                85                  90


<210>  15
<211>  36
<212>  DNA
<213>  artificial sequence

<220>
<223>  peptide linker

<220>
<221>  CDS
<222>  (1)..(36)
<223>

<400>  15
gct agc ccc agc aac ccc ggc gcc agc aac gga tcc                        36
Ala Ser Pro Ser Asn Pro Gly Ala Ser Asn Gly Ser
1               5                   10


<210>  16
<211>  12
<212>  PRT
<213>  artificial sequence

<220>
<223>  peptide linker

<400>  16
Ala Ser Pro Ser Asn Pro Gly Ala Ser Asn Gly Ser
1               5                   10


<210>  17
<211>  42
<212>  DNA
<213>  artificial sequence

<220>
<223>  peptide linker

<220>
<221>  CDS
<222>  (1)..(42)
<223>

<400>  17
gct agc ggc gga gga agc gga ggc gga agc gga gga gga tcc                42
Ala Ser Gly Gly Gly Ser Gly Gly Gly Ser Gly Gly Gly Ser
1               5                   10


<210>  18
<211>  14
<212>  PRT
<213>  artificial sequence

<220>
<223>  peptide linker

<400>  18
Ala Ser Gly Gly Gly Ser Gly Gly Gly Ser Gly Gly Gly Ser
1               5                   10


<210>  19
<211>  57
<212>  DNA
<213>  artificial sequence

<220>
<223>  peptide linker

<220>
<221>  CDS
<222>  (1)..(57)
<223>

<400>  19
gct agc ggc gcc gag gcc gct gcc aag gag gcc gct gcc aag gcc gga        48
Ala Ser Gly Ala Glu Ala Ala Ala Lys Glu Ala Ala Ala Lys Ala Gly
1               5                   10                  15

ggc gga tcc                                                            57
Gly Gly Ser


<210>  20
<211>  19
<212>  PRT
<213>  artificial sequence

<220>
<223>  peptide linker

<400>  20
Ala Ser Gly Ala Glu Ala Ala Ala Lys Glu Ala Ala Ala Lys Ala Gly
1               5                   10                  15

Gly Gly Ser


<210>  21
<211>  18
<212>  DNA
<213>  artificial sequence

<220>
<223>  peptide linker

<220>
<221>  CDS
<222>  (1)..(18)
<223>

<400>  21

gct agc acc ggt gga tcc                                                    18
Ala Ser Thr Gly Gly Ser
1               5


<210>  22
<211>  6
<212>  PRT
<213>  artificial sequence

<220>
<223>  peptide linker

<400>  22
Ala Ser Thr Gly Gly Ser
1               5


<210>  23
<211>  51
<212>  DNA
<213>  artificial sequence

<220>
<223>  peptide linker

<220>
<221>  CDS
<222>  (1)..(51)
<223>

<400>  23
gct agc gga ggc agc ggc gga gga agc gga ggc gga agc gga gga gga        48
Ala Ser Gly Gly Ser Gly Gly Gly Ser Gly Gly Gly Ser Gly Gly Gly
1               5                   10                  15

tcc                                                                    51
Ser


<210>  24
<211>  17
<212>  PRT
<213>  artificial sequence

<220>
<223>  peptide linker

<400>  24
Ala Ser Gly Gly Ser Gly Gly Gly Ser Gly Gly Gly Ser Gly Gly Gly
1               5                   10                  15

Ser


<210>  25
<211>  51
<212>  DNA

<213>  artificial sequence

<220>
<223>  peptide linker

<220>
<221>  CDS
<222>  (1)..(51)
<223>

<400>  25
gct agc gga ggc agc ggc gga gga agc gga ggc gga agc gga gga gga        48
Ala Ser Gly Gly Ser Gly Gly Gly Ser Gly Gly Gly Ser Gly Gly Gly
1               5                   10                  15

tcc                                                                    51
Ser

<210>  26
<211>  17
<212>  PRT
<213>  artificial sequence

<220>
<223>  peptide linker

<400>  26
Ala Ser Gly Gly Ser Gly Gly Gly Ser Gly Gly Gly Ser Gly Gly Gly
1               5                   10                  15

Ser

<210>  27
<211>  48
<212>  DNA
<213>  artificial sequence

<220>
<223>  peptide linker

<220>
<221>  CDS
<222>  (1)..(48)
<223>

<400>  27
gct agc gcc gag gcc gct gcc aag gag gcc gct gcc aag gcc gga tcc        48
Ala Ser Ala Glu Ala Ala Ala Lys Glu Ala Ala Ala Lys Ala Gly Ser
1               5                   10                  15

<210>  28
<211>  16
<212>  PRT
<213>  artificial sequence

<220>
<223>  peptide linker

<400>  28
Ala Ser Ala Glu Ala Ala Ala Lys Glu Ala Ala Ala Lys Ala Gly Ser
1               5                   10                  15


<210>  29
<211>  63
<212>  DNA
<213>  artificial sequence

<220>
<223>  peptide linker

<220>
<221>  CDS
<222>  (1)..(63)
<223>

<400>  29
gct agc gcc gag gct gcc gcc aag gaa gcc gct gcc aag gaa gcc gcc       48
Ala Ser Ala Glu Ala Ala Ala Lys Glu Ala Ala Ala Lys Glu Ala Ala
1               5                   10                  15

gct aaa gct gga tcc                                                   63
Ala Lys Ala Gly Ser
            20


<210>  30
<211>  21
<212>  PRT
<213>  artificial sequence

<220>
<223>  peptide linker

<400>  30
Ala Ser Ala Glu Ala Ala Ala Lys Glu Ala Ala Ala Lys Glu Ala Ala
1               5                   10                  15

Ala Lys Ala Gly Ser
            20


<210>  31
<211>  60
<212>  DNA
<213>  artificial sequence

<220>
<223>  peptide linker

<220>
<221>  CDS
<222>  (1)..(60)
<223>

<400>  31

gct agc gga ggc agc ggc gga gga agc gga ggc gga agc gga gga tct 48
Ala Ser Gly Gly Ser Gly Gly Gly Ser Gly Gly Gly Ser Gly Gly Ser
1               5                   10                  15

ggc gga gga tcc 60
Gly Gly Gly Ser
            20

<210> 32
<211> 20
<212> PRT
<213> artificial sequence

<220>
<223> peptide linker

<400> 32
Ala Ser Gly Gly Ser Gly Gly Gly Ser Gly Gly Gly Ser Gly Gly Ser
1               5                   10                  15

Gly Gly Gly Ser
            20

<210> 33
<211> 69
<212> DNA
<213> artificial sequence

<220>
<223> peptide linker

<220>
<221> CDS
<222> (1)..(69)
<223>

<400> 33
gct agc gga ggc agc ggc gga gga agc gga ggc gga agc gga gga tct 48
Ala Ser Gly Gly Ser Gly Gly Gly Ser Gly Gly Gly Ser Gly Gly Ser
1               5                   10                  15

ggc gga tcc ggc ggc gga tcc 69
Gly Gly Ser Gly Gly Gly Ser
            20

<210> 34
<211> 23
<212> PRT
<213> artificial sequence

<220>
<223> peptide linker

<400> 34
Ala Ser Gly Gly Ser Gly Gly Gly Ser Gly Gly Gly Ser Gly Gly Ser
1               5                   10                  15

Gly Gly Ser Gly Gly Gly Ser
            20

<210>  35
<211>  78
<212>  DNA
<213>  artificial sequence

<220>
<223>  peptide linker

<220>
<221>  CDS
<222>  (1)..(78)
<223>

<400>  35
gct agc gcc gag gcc gcc gct aag gag gct gcc gcc aag gaa gcc gct        48
Ala Ser Ala Glu Ala Ala Ala Lys Glu Ala Ala Ala Lys Glu Ala Ala
1               5                   10                  15

gcc aag gaa gcc gcc gct aaa gct gga tcc                                78
Ala Lys Glu Ala Ala Ala Lys Ala Gly Ser
            20                  25

<210>  36
<211>  26
<212>  PRT
<213>  artificial sequence

<220>
<223>  peptide linker

<400>  36
Ala Ser Ala Glu Ala Ala Ala Lys Glu Ala Ala Ala Lys Glu Ala Ala
1               5                   10                  15

Ala Lys Glu Ala Ala Ala Lys Ala Gly Ser
            20                  25

<210>  37
<211>  93
<212>  DNA
<213>  artificial sequence

<220>
<223>  peptide linker

<220>
<221>  CDS
<222>  (1)..(93)
<223>

<400>  37
gct agc gcc gag gcc gcc gct aag gaa gct gct gcc aaa gag gct gcc        48
Ala Ser Ala Glu Ala Ala Ala Lys Glu Ala Ala Ala Lys Glu Ala Ala
1               5                   10                  15

gcc aag gaa gcc gct gcc aag gaa gcc gcc gct aaa gct gga tcc          93
Ala Lys Glu Ala Ala Ala Lys Glu Ala Ala Ala Lys Ala Gly Ser
            20                  25                  30

<210>  38
<211>  31
<212>  PRT
<213>  artificial sequence

<220>
<223>  peptide linker

<400>  38
Ala Ser Ala Glu Ala Ala Ala Lys Glu Ala Ala Ala Lys Glu Ala Ala
1               5                   10                  15

Ala Lys Glu Ala Ala Ala Lys Glu Ala Ala Ala Lys Ala Gly Ser
            20                  25                  30

<210>  39
<211>  27
<212>  DNA
<213>  artificial sequence

<220>
<223>  nuclear localization signal

<220>
<221>  CDS
<222>  (1)..(27)
<223>

<400>  39
atg gcc cct aag aag aag aga aag gtg                                  27
Met Ala Pro Lys Lys Lys Arg Lys Val
1               5

<210>  40
<211>  9
<212>  PRT
<213>  artificial sequence

<220>
<223>  nuclear localization signal

<400>  40
Met Ala Pro Lys Lys Lys Arg Lys Val
1               5

<210>  41
<211>  10
<212>  DNA
<213>  artificial sequence

<220>
<223>  Kozak sequence

<220>
<221> CDS
<222> (7)..(9)
<223>

<400> 41
gccacc atg g 10
Met
1

<210> 42
<211> 1
<212> PRT
<213> artificial sequence

<220>
<223> Kozak sequence

<400> 42
Met
1

<210> 43
<211> 624

<212> DNA
<213> artificial sequence

<220>
<223> polypeptide comprising SEQ ID NO : 42, SEQ ID NO : 40,
SEQ ID NO : 12, a peptide linker chosen among SEQ ID NO : 16
to SEQ ID NO : 38, and SEQ ID NO : 4

<220>
<221> CDS
<222> (7)..(621)
<223>

<220>
<221> misc_feature
<222> (361)..(363)
<223> SEQ ID NO : 15 to SEQ ID NO 37

<400> 43
gccacc atg gcc cct aag aag aag aga aag gtg tct aga gga gtg cag 48
Met Ala Pro Lys Lys Lys Arg Lys Val Ser Arg Gly Val Gln
1               5                   10

gtg gaa acc atc tcc cca gga gac ggg cgc acc ttc ccc aag cgc ggc 96
Val Glu Thr Ile Ser Pro Gly Asp Gly Arg Thr Phe Pro Lys Arg Gly
15                  20                  25                  30

cag acc tgc gtg gtg cac tac acc ggg atg ctt gaa gat gga aag aaa 144
Gln Thr Cys Val Val His Tyr Thr Gly Met Leu Glu Asp Gly Lys Lys
                35                  40                  45

ttt gat tcc tcc cgg gac aga aac aag ccc ttt aag ttt atg cta ggc 192
Phe Asp Ser Ser Arg Asp Arg Asn Lys Pro Phe Lys Phe Met Leu Gly
            50                  55                  60

aag cag gag gtg atc cga ggc tgg gaa gaa ggg gtt gcc cag atg agt      240
Lys Gln Glu Val Ile Arg Gly Trp Glu Glu Gly Val Ala Gln Met Ser
        65                  70                  75

gtg ggt cag aga gcc aaa ctg act ata tct cca gat tat gcc tat ggt      288
Val Gly Gln Arg Ala Lys Leu Thr Ile Ser Pro Asp Tyr Ala Tyr Gly
    80                  85                  90

gcc act ggg cac cca ggc atc atc cca cca cat gcc act ctc gtc ttc      336
Ala Thr Gly His Pro Gly Ile Ile Pro Pro His Ala Thr Leu Val Phe
95                  100                 105                 110

gat gtg gag ctt cta aaa ctg gaa nnn acc tct gat gaa gtc agg aag      384
Asp Val Glu Leu Leu Lys Leu Glu Xaa Thr Ser Asp Glu Val Arg Lys
                115                 120                 125

aac ctg atg gac atg ttc agg gac agg cag gcc ttc tct gaa cac acc      432
Asn Leu Met Asp Met Phe Arg Asp Arg Gln Ala Phe Ser Glu His Thr
            130                 135                 140

tgg aag atg ctc ctg tct gtg tgc aga tcc tgg gct gcc tgg tgc aag      480
Trp Lys Met Leu Leu Ser Val Cys Arg Ser Trp Ala Ala Trp Cys Lys
        145                 150                 155

ctg aac aac agg aaa tgg ttc cct gct gaa cct gag gat gtg agg gac      528
Leu Asn Asn Arg Lys Trp Phe Pro Ala Glu Pro Glu Asp Val Arg Asp
    160                 165                 170

tac ctc ctg tac ctg caa gcc aga ggc ctg gct gtg aag acc atc caa      576
Tyr Leu Leu Tyr Leu Gln Ala Arg Gly Leu Ala Val Lys Thr Ile Gln
175                 180                 185                 190

cag cac ctg ggc cag ctc aac atg ctg cac agg aga tct ggc ctg tga      624
Gln His Leu Gly Gln Leu Asn Met Leu His Arg Arg Ser Gly Leu
                195                 200                 205


<210>  44
<211>  205
<212>  PRT
<213>  artificial sequence

<220>
<221>  misc_feature
<222>  (119)..(119)
<223>  The 'Xaa' at location 119 stands for SEQ ID NO : 16
       to SEQ ID NO : 38

<220>
<223>  polypeptide comprising SEQ ID NO : 42, SEQ ID NO : 40,
       SEQ ID NO : 12, a peptide linker chosen among SEQ ID NO : 16
       to SEQ ID NO : 38, and SEQ ID NO : 4

<220>
<221>  misc_feature
<222>  (361)..(363)
<223>  SEQ ID NO : 15 to SEQ ID NO 37

<400> 44
Met Ala Pro Lys Lys Lys Arg Lys Val Ser Arg Gly Val Gln Val Glu
1               5                   10                  15

Thr Ile Ser Pro Gly Asp Gly Arg Thr Phe Pro Lys Arg Gly Gln Thr
            20                  25                  30

Cys Val Val His Tyr Thr Gly Met Leu Glu Asp Gly Lys Lys Phe Asp
        35                  40                  45

Ser Ser Arg Asp Arg Asn Lys Pro Phe Lys Phe Met Leu Gly Lys Gln
    50                  55                  60

Glu Val Ile Arg Gly Trp Glu Glu Gly Val Ala Gln Met Ser Val Gly
65                  70                  75                  80

Gln Arg Ala Lys Leu Thr Ile Ser Pro Asp Tyr Ala Tyr Gly Ala Thr
                85                  90                  95

Gly His Pro Gly Ile Ile Pro Pro His Ala Thr Leu Val Phe Asp Val
            100                 105                 110

Glu Leu Leu Lys Leu Glu Xaa Thr Ser Asp Glu Val Arg Lys Asn Leu
        115                 120                 125

Met Asp Met Phe Arg Asp Arg Gln Ala Phe Ser Glu His Thr Trp Lys
    130                 135                 140

Met Leu Leu Ser Val Cys Arg Ser Trp Ala Ala Trp Cys Lys Leu Asn
145                 150                 155                 160

Asn Arg Lys Trp Phe Pro Ala Glu Pro Glu Asp Val Arg Asp Tyr Leu
                165                 170                 175

Leu Tyr Leu Gln Ala Arg Gly Leu Ala Val Lys Thr Ile Gln Gln His
            180                 185                 190

Leu Gly Gln Leu Asn Met Leu His Arg Arg Ser Gly Leu
        195                 200                 205


<210> 45
<211> 489
<212> DNA
<213> artificial sequence

<220>
<223> polypeptide comprising SEQ ID NO : 42, SEQ ID NO : 40,
      SEQ ID NO : 12, a peptide linker chosen among SEQ ID NO : 16
      to SEQ ID NO : 38, and SEQ ID NO : 6

<220>
<221> CDS
<222> (7)..(486)
<223>

<220>
<221> misc_feature
<222> (361)..(363)
<223> SEQ ID NO 15 to SEQ ID NO 37

<400> 45
gccacc atg gcc cct aag aag aag aga aag gtg tct aga gga gtg cag 48
Met Ala Pro Lys Lys Lys Arg Lys Val Ser Arg Gly Val Gln
1               5                   10

gtg gaa acc atc tcc cca gga gac ggg cgc acc ttc ccc aag cgc ggc 96
Val Glu Thr Ile Ser Pro Gly Asp Gly Arg Thr Phe Pro Lys Arg Gly
15                  20                  25                  30

cag acc tgc gtg gtg cac tac acc ggg atg ctt gaa gat gga aag aaa 144
Gln Thr Cys Val Val His Tyr Thr Gly Met Leu Glu Asp Gly Lys Lys
                35                  40                  45

ttt gat tcc tcc cgg gac aga aac aag ccc ttt aag ttt atg cta ggc 192
Phe Asp Ser Ser Arg Asp Arg Asn Lys Pro Phe Lys Phe Met Leu Gly
            50                  55                  60

aag cag gag gtg atc cga ggc tgg gaa gaa ggg gtt gcc cag atg agt 240
Lys Gln Glu Val Ile Arg Gly Trp Glu Glu Gly Val Ala Gln Met Ser
        65                  70                  75

gtg ggt cag aga gcc aaa ctg act ata tct cca gat tat gcc tat ggt 288
Val Gly Gln Arg Ala Lys Leu Thr Ile Ser Pro Asp Tyr Ala Tyr Gly
    80                  85                  90

gcc act ggg cac cca ggc atc atc cca cca cat gcc act ctc gtc ttc 336
Ala Thr Gly His Pro Gly Ile Ile Pro Pro His Ala Thr Leu Val Phe
95                  100                 105                 110

gat gtg gag ctt cta aaa ctg gaa nnn acc tct gat gaa gtc agg aag 384
Asp Val Glu Leu Leu Lys Leu Glu Xaa Thr Ser Asp Glu Val Arg Lys
                115                 120                 125

aac ctg atg gac atg ttc agg gac agg cag gcc ttc tct gaa cac acc 432
Asn Leu Met Asp Met Phe Arg Asp Arg Gln Ala Phe Ser Glu His Thr
            130                 135                 140

tgg aag atg ctc ctg tct gtg tgc aga tcc tgg gct gcc tgg tgc aag 480
Trp Lys Met Leu Leu Ser Val Cys Arg Ser Trp Ala Ala Trp Cys Lys
        145                 150                 155

ctg aac tga 489
Leu Asn
    160


<210> 46
<211> 160
<212> PRT
<213> artificial sequence

<220>
<221> misc_feature
<222> (119)..(119)
<223> The 'Xaa' at location 119 stands for SEQ ID NO : 16
to SEQ ID NO : 38

<220>
<223> polypeptide comprising SEQ ID NO : 42, SEQ ID NO : 40,
SEQ ID NO : 12, a peptide linker chosen among SEQ ID NO : 16

to SEQ ID NO : 38, and SEQ ID NO : 6

<220>
<221> misc_feature
<222> (361)..(363)
<223> SEQ ID NO 15 to SEQ ID NO 37

<400> 46
Met Ala Pro Lys Lys Lys Arg Lys Val Ser Arg Gly Val Gln Val Glu
1               5                   10                  15

Thr Ile Ser Pro Gly Asp Gly Arg Thr Phe Pro Lys Arg Gly Gln Thr
            20                  25                  30

Cys Val Val His Tyr Thr Gly Met Leu Glu Asp Gly Lys Lys Phe Asp
        35                  40                  45

Ser Ser Arg Asp Arg Asn Lys Pro Phe Lys Phe Met Leu Gly Lys Gln
    50                  55                  60

Glu Val Ile Arg Gly Trp Glu Glu Gly Val Ala Gln Met Ser Val Gly
65                  70                  75                  80

Gln Arg Ala Lys Leu Thr Ile Ser Pro Asp Tyr Ala Tyr Gly Ala Thr
                85                  90                  95

Gly His Pro Gly Ile Ile Pro Pro His Ala Thr Leu Val Phe Asp Val
            100                 105                 110

Glu Leu Leu Lys Leu Glu Xaa Thr Ser Asp Glu Val Arg Lys Asn Leu
        115                 120                 125

Met Asp Met Phe Arg Asp Arg Gln Ala Phe Ser Glu His Thr Trp Lys
    130                 135                 140

Met Leu Leu Ser Val Cys Arg Ser Trp Ala Ala Trp Cys Lys Leu Asn
145                 150                 155                 160


<210> 47
<211> 1035
<212> DNA
<213> artificial sequence

<220>
<223> polypeptide comprising SEQ ID NO : 42, SEQ ID NO : 40,
SEQ ID NO : 14, a peptide linker chosen among SEQ ID NO : 16
to SEQ ID NO : 38, and SEQ ID NO : 8


<220>
<221> CDS
<222> (7)..(1032)
<223>

<220>
<221> misc_feature
<222> (316)..(318)
<223> SEQ ID NO 15 to SEQ ID NO 37

<400> 47
gccacc atg gcc cct aag aag aag aga aag gtg tct aga atc ctc tgg 48
Met Ala Pro Lys Lys Lys Arg Lys Val Ser Arg Ile Leu Trp
1               5                   10

cat gag atg tgg cat gaa ggc ctg gaa gag gca tct cgt ttg tac ttt 96
His Glu Met Trp His Glu Gly Leu Glu Glu Ala Ser Arg Leu Tyr Phe
15                  20                  25                  30

ggg gaa agg aac gtg aaa ggc atg ttt gag gtg ctg gag ccc ttg cat 144
Gly Glu Arg Asn Val Lys Gly Met Phe Glu Val Leu Glu Pro Leu His
                35                  40                  45

gct atg atg gaa cgg ggc ccc cag act ctg aag gaa aca tcc ttt aat 192
Ala Met Met Glu Arg Gly Pro Gln Thr Leu Lys Glu Thr Ser Phe Asn
            50                  55                  60

cag gcc tat ggt cga gat tta atg gag gcc caa gag tgg tgc agg aag 240
Gln Ala Tyr Gly Arg Asp Leu Met Glu Ala Gln Glu Trp Cys Arg Lys
        65                  70                  75

tac atg aaa tca ggg aat gtc aag gac ctc ctc caa gcc tgg gac ctc 288
Tyr Met Lys Ser Gly Asn Val Lys Asp Leu Leu Gln Ala Trp Asp Leu
    80                  85                  90

tat tat cat gtg ttc cga cga atc tca nnn cgc cct tct gac tcc aat 336
Tyr Tyr His Val Phe Arg Arg Ile Ser Xaa Arg Pro Ser Asp Ser Asn
95                  100                 105                 110

gct gtg tcc ctg gtg atg agg aga atc aga aag gag aat gtg gat gct 384
Ala Val Ser Leu Val Met Arg Arg Ile Arg Lys Glu Asn Val Asp Ala
                115                 120                 125

ggg gag aga gcc aag cag gcc ctg gcc ttt gaa cgc act gac ttt gac 432
Gly Glu Arg Ala Lys Gln Ala Leu Ala Phe Glu Arg Thr Asp Phe Asp
            130                 135                 140

caa gtc aga tcc ctg atg gag aac tct gac aga tgc cag gac atc agg 480
Gln Val Arg Ser Leu Met Glu Asn Ser Asp Arg Cys Gln Asp Ile Arg
        145                 150                 155

aac ctg gcc ttc ctg ggc att gcc tac aac acc ctg ctg cgc att gcc 528
Asn Leu Ala Phe Leu Gly Ile Ala Tyr Asn Thr Leu Leu Arg Ile Ala
    160                 165                 170

gaa att gcc aga atc aga gtg aag gac atc tcc cgc acc gat ggt ggg 576
Glu Ile Ala Arg Ile Arg Val Lys Asp Ile Ser Arg Thr Asp Gly Gly
175                 180                 185                 190

aga atg ctg atc cac att ggc agg acc aag acc ctg gtg tcc aca gct 624
Arg Met Leu Ile His Ile Gly Arg Thr Lys Thr Leu Val Ser Thr Ala
                195                 200                 205

ggt gtg gag aag gcc ctg tcc ctg ggg gtt acc aag ctg gtg gag aga 672
Gly Val Glu Lys Ala Leu Ser Leu Gly Val Thr Lys Leu Val Glu Arg
            210                 215                 220

tgg atc tct gtg tct ggt gtg gct gat gac ccc aac aac tac ctg ttc 720
Trp Ile Ser Val Ser Gly Val Ala Asp Asp Pro Asn Asn Tyr Leu Phe
        225                 230                 235

tgc cgg gtc aga aag aat ggt gtg gct gcc cct tct gcc acc tcc caa     768
Cys Arg Val Arg Lys Asn Gly Val Ala Ala Pro Ser Ala Thr Ser Gln
    240                 245                 250

ctg tcc acc cgg gcc ctg gaa ggg atc ttt gag gcc acc cac cgc ctg     816
Leu Ser Thr Arg Ala Leu Glu Gly Ile Phe Glu Ala Thr His Arg Leu
255                 260                 265                 270

atc tat ggt gcc aag gat gac tct ggg cag aga tac ctg gcc tgg tct     864
Ile Tyr Gly Ala Lys Asp Asp Ser Gly Gln Arg Tyr Leu Ala Trp Ser
                275                 280                 285

ggc cac tct gcc aga gtg ggt gct gcc agg gac atg gcc agg gct ggt     912
Gly His Ser Ala Arg Val Gly Ala Ala Arg Asp Met Ala Arg Ala Gly
            290                 295                 300

gtg tcc atc cct gaa atc atg cag gct ggt ggc tgg acc aat gtg aac     960
Val Ser Ile Pro Glu Ile Met Gln Ala Gly Gly Trp Thr Asn Val Asn
        305                 310                 315

att gtg atg aac tac atc aga aac ctg gac tct gag act ggg gcc atg    1008
Ile Val Met Asn Tyr Ile Arg Asn Leu Asp Ser Glu Thr Gly Ala Met
    320                 325                 330

gtg agg ctg ctc gag gat ggg gac tga                                1035
Val Arg Leu Leu Glu Asp Gly Asp
335                 340

<210>  48
<211>  342
<212>  PRT
<213>  artificial sequence

<220>
<221>  misc_feature
<222>  (104)..(104)
<223>  The 'Xaa' at location 104 stands for SEQ ID NO : 16
       to SEQ ID NO : 38

<220>
<223>  polypeptide comprising SEQ ID NO : 42, SEQ ID NO : 40,
       SEQ ID NO : 14, a peptide linker chosen among SEQ ID NO : 16
       to SEQ ID NO : 38, and SEQ ID NO : 8

<220>
<221>  misc_feature
<222>  (316)..(318)
<223>  SEQ ID NO 15 to SEQ ID NO 37

<400>  48
Met Ala Pro Lys Lys Lys Arg Lys Val Ser Arg Ile Leu Trp His Glu
1               5                   10                  15

Met Trp His Glu Gly Leu Glu Glu Ala Ser Arg Leu Tyr Phe Gly Glu
            20                  25                  30

Arg Asn Val Lys Gly Met Phe Glu Val Leu Glu Pro Leu His Ala Met
        35                  40                  45

Met Glu Arg Gly Pro Gln Thr Leu Lys Glu Thr Ser Phe Asn Gln Ala
    50                  55                  60

Tyr Gly Arg Asp Leu Met Glu Ala Gln Glu Trp Cys Arg Lys Tyr Met
65                  70                  75                  80

Lys Ser Gly Asn Val Lys Asp Leu Leu Gln Ala Trp Asp Leu Tyr Tyr
                85                  90                  95

His Val Phe Arg Arg Ile Ser Xaa Arg Pro Ser Asp Ser Asn Ala Val
            100                 105                 110

Ser Leu Val Met Arg Arg Ile Arg Lys Glu Asn Val Asp Ala Gly Glu
        115                 120                 125

Arg Ala Lys Gln Ala Leu Ala Phe Glu Arg Thr Asp Phe Asp Gln Val
    130                 135                 140

Arg Ser Leu Met Glu Asn Ser Asp Arg Cys Gln Asp Ile Arg Asn Leu
145                 150                 155                 160

Ala Phe Leu Gly Ile Ala Tyr Asn Thr Leu Leu Arg Ile Ala Glu Ile
                165                 170                 175

Ala Arg Ile Arg Val Lys Asp Ile Ser Arg Thr Asp Gly Gly Arg Met
            180                 185                 190

Leu Ile His Ile Gly Arg Thr Lys Thr Leu Val Ser Thr Ala Gly Val
        195                 200                 205

Glu Lys Ala Leu Ser Leu Gly Val Thr Lys Leu Val Glu Arg Trp Ile
    210                 215                 220

Ser Val Ser Gly Val Ala Asp Asp Pro Asn Asn Tyr Leu Phe Cys Arg
225                 230                 235                 240

Val Arg Lys Asn Gly Val Ala Ala Pro Ser Ala Thr Ser Gln Leu Ser
                245                 250                 255

Thr Arg Ala Leu Glu Gly Ile Phe Glu Ala Thr His Arg Leu Ile Tyr
            260                 265                 270

Gly Ala Lys Asp Asp Ser Gly Gln Arg Tyr Leu Ala Trp Ser Gly His
        275                 280                 285

Ser Ala Arg Val Gly Ala Ala Arg Asp Met Ala Arg Ala Gly Val Ser
    290                 295                 300

Ile Pro Glu Ile Met Gln Ala Gly Gly Trp Thr Asn Val Asn Ile Val
305                 310                 315                 320

Met Asn Tyr Ile Arg Asn Leu Asp Ser Glu Thr Gly Ala Met Val Arg
                325                 330                 335

Leu Leu Glu Asp Gly Asp
            340

<210>  49
<211>  1173
<212>  DNA

<213>  artificial sequence

<220>
<223>  polypeptide comprising SEQ ID NO : 42, SEQ ID NO : 40,
       SEQ ID NO : 14, a peptide linker chosen among SEQ ID NO : 16
       to SEQ ID NO : 38, and SEQ ID NO : 10

<220>
<221>  CDS
<222>  (7)..(1170)
<223>

<220>
<221>  misc_feature
<222>  (316)..(318)
<223>  SEQ ID NO 15 to SEQ ID NO 37

<400>  49
gccacc atg gcc cct aag aag aag aga aag gtg tct aga atc ctc tgg        48
       Met Ala Pro Lys Lys Lys Arg Lys Val Ser Arg Ile Leu Trp
       1               5                   10

cat gag atg tgg cat gaa ggc ctg gaa gag gca tct cgt ttg tac ttt       96
His Glu Met Trp His Glu Gly Leu Glu Glu Ala Ser Arg Leu Tyr Phe
15                  20                  25                  30

ggg gaa agg aac gtg aaa ggc atg ttt gag gtg ctg gag ccc ttg cat      144
Gly Glu Arg Asn Val Lys Gly Met Phe Glu Val Leu Glu Pro Leu His
                35                  40                  45

gct atg atg gaa cgg ggc ccc cag act ctg aag gaa aca tcc ttt aat      192
Ala Met Met Glu Arg Gly Pro Gln Thr Leu Lys Glu Thr Ser Phe Asn
            50                  55                  60

cag gcc tat ggt cga gat tta atg gag gcc caa gag tgg tgc agg aag      240
Gln Ala Tyr Gly Arg Asp Leu Met Glu Ala Gln Glu Trp Cys Arg Lys
        65                  70                  75

tac atg aaa tca ggg aat gtc aag gac ctc ctc caa gcc tgg gac ctc      288
Tyr Met Lys Ser Gly Asn Val Lys Asp Leu Leu Gln Ala Trp Asp Leu
    80                  85                  90

tat tat cat gtg ttc cga cga atc tca nnn aac agg aaa tgg ttc cct      336
Tyr Tyr His Val Phe Arg Arg Ile Ser Xaa Asn Arg Lys Trp Phe Pro
95                  100                 105                 110

gct gaa cct gag gat gtg agg gac tac ctc ctg tac ctg caa gcc aga      384
Ala Glu Pro Glu Asp Val Arg Asp Tyr Leu Leu Tyr Leu Gln Ala Arg
                115                 120                 125

ggc ctg gct gtg aag acc atc caa cag cac ctg ggc cag ctc aac atg      432
Gly Leu Ala Val Lys Thr Ile Gln Gln His Leu Gly Gln Leu Asn Met
            130                 135                 140

ctg cac agg aga tct ggc ctg cct cgc cct tct gac tcc aat gct gtg      480
Leu His Arg Arg Ser Gly Leu Pro Arg Pro Ser Asp Ser Asn Ala Val
        145                 150                 155

tcc ctg gtg atg agg aga atc aga aag gag aat gtg gat gct ggg gag      528
Ser Leu Val Met Arg Arg Ile Arg Lys Glu Asn Val Asp Ala Gly Glu
    160                 165                 170

aga gcc aag cag gcc ctg gcc ttt gaa cgc act gac ttt gac caa gtc 576
Arg Ala Lys Gln Ala Leu Ala Phe Glu Arg Thr Asp Phe Asp Gln Val
175                 180                 185                 190

aga tcc ctg atg gag aac tct gac aga tgc cag gac atc agg aac ctg 624
Arg Ser Leu Met Glu Asn Ser Asp Arg Cys Gln Asp Ile Arg Asn Leu
                195                 200                 205

gcc ttc ctg ggc att gcc tac aac acc ctg ctg cgc att gcc gaa att 672
Ala Phe Leu Gly Ile Ala Tyr Asn Thr Leu Leu Arg Ile Ala Glu Ile
            210                 215                 220

gcc aga atc aga gtg aag gac atc tcc cgc acc gat ggt ggg aga atg 720
Ala Arg Ile Arg Val Lys Asp Ile Ser Arg Thr Asp Gly Gly Arg Met
        225                 230                 235

ctg atc cac att ggc agg acc aag acc ctg gtg tcc aca gct ggt gtg 768
Leu Ile His Ile Gly Arg Thr Lys Thr Leu Val Ser Thr Ala Gly Val
    240                 245                 250

gag aag gcc ctg tcc ctg ggg gtt acc aag ctg gtg gag aga tgg atc 816
Glu Lys Ala Leu Ser Leu Gly Val Thr Lys Leu Val Glu Arg Trp Ile
255                 260                 265                 270

tct gtg tct ggt gtg gct gat gac ccc aac aac tac ctg ttc tgc cgg 864
Ser Val Ser Gly Val Ala Asp Asp Pro Asn Asn Tyr Leu Phe Cys Arg
                275                 280                 285

gtc aga aag aat ggt gtg gct gcc cct tct gcc acc tcc caa ctg tcc 912
Val Arg Lys Asn Gly Val Ala Ala Pro Ser Ala Thr Ser Gln Leu Ser
            290                 295                 300

acc cgg gcc ctg gaa ggg atc ttt gag gcc acc cac cgc ctg atc tat 960
Thr Arg Ala Leu Glu Gly Ile Phe Glu Ala Thr His Arg Leu Ile Tyr
        305                 310                 315

ggt gcc aag gat gac tct ggg cag aga tac ctg gcc tgg tct ggc cac 1008
Gly Ala Lys Asp Asp Ser Gly Gln Arg Tyr Leu Ala Trp Ser Gly His
    320                 325                 330

tct gcc aga gtg ggt gct gcc agg gac atg gcc agg gct ggt gtg tcc 1056
Ser Ala Arg Val Gly Ala Ala Arg Asp Met Ala Arg Ala Gly Val Ser
335                 340                 345                 350

atc cct gaa atc atg cag gct ggt ggc tgg acc aat gtg aac att gtg 1104
Ile Pro Glu Ile Met Gln Ala Gly Gly Trp Thr Asn Val Asn Ile Val
                355                 360                 365

atg aac tac atc aga aac ctg gac tct gag act ggg gcc atg gtg agg 1152
Met Asn Tyr Ile Arg Asn Leu Asp Ser Glu Thr Gly Ala Met Val Arg
            370                 375                 380

ctg ctc gag gat ggg gac tga 1173
Leu Leu Glu Asp Gly Asp
        385

<210> 50
<211> 388
<212> PRT

<213>  artificial sequence

<220>
<221>  misc_feature
<222>  (104)..(104)
<223>  The 'Xaa' at location 104 stands for SEQ ID NO : 16
      to SEQ ID NO : 38

<220>
<223>  polypeptide comprising SEQ ID NO : 42, SEQ ID NO : 40,
      SEQ ID NO : 14, a peptide linker chosen among SEQ ID NO : 16
      to SEQ ID NO : 38, and SEQ ID NO : 10

<220>
<221>  misc_feature
<222>  (316)..(318)
<223>  SEQ ID NO 15 to SEQ ID NO 37

<400>  50
Met Ala Pro Lys Lys Lys Arg Lys Val Ser Arg Ile Leu Trp His Glu
1               5                   10                  15

Met Trp His Glu Gly Leu Glu Glu Ala Ser Arg Leu Tyr Phe Gly Glu
            20                  25                  30

Arg Asn Val Lys Gly Met Phe Glu Val Leu Glu Pro Leu His Ala Met
        35                  40                  45

Met Glu Arg Gly Pro Gln Thr Leu Lys Glu Thr Ser Phe Asn Gln Ala
    50                  55                  60

Tyr Gly Arg Asp Leu Met Glu Ala Gln Glu Trp Cys Arg Lys Tyr Met
65                  70                  75                  80

Lys Ser Gly Asn Val Lys Asp Leu Leu Gln Ala Trp Asp Leu Tyr Tyr
                85                  90                  95

His Val Phe Arg Arg Ile Ser Xaa Asn Arg Lys Trp Phe Pro Ala Glu
            100                 105                 110

Pro Glu Asp Val Arg Asp Tyr Leu Leu Tyr Leu Gln Ala Arg Gly Leu
        115                 120                 125

Ala Val Lys Thr Ile Gln Gln His Leu Gly Gln Leu Asn Met Leu His
    130                 135                 140

Arg Arg Ser Gly Leu Pro Arg Pro Ser Asp Ser Asn Ala Val Ser Leu
145                 150                 155                 160

Val Met Arg Arg Ile Arg Lys Glu Asn Val Asp Ala Gly Glu Arg Ala
                165                 170                 175

Lys Gln Ala Leu Ala Phe Glu Arg Thr Asp Phe Asp Gln Val Arg Ser
            180                 185                 190

Leu Met Glu Asn Ser Asp Arg Cys Gln Asp Ile Arg Asn Leu Ala Phe
        195                 200                 205

Leu Gly Ile Ala Tyr Asn Thr Leu Leu Arg Ile Ala Glu Ile Ala Arg
    210                 215                 220

Ile Arg Val Lys Asp Ile Ser Arg Thr Asp Gly Gly Arg Met Leu Ile
225                 230                 235                 240

His Ile Gly Arg Thr Lys Thr Leu Val Ser Thr Ala Gly Val Glu Lys
                245                 250                 255

Ala Leu Ser Leu Gly Val Thr Lys Leu Val Glu Arg Trp Ile Ser Val
            260                 265                 270

Ser Gly Val Ala Asp Asp Pro Asn Asn Tyr Leu Phe Cys Arg Val Arg
        275                 280                 285

Lys Asn Gly Val Ala Ala Pro Ser Ala Thr Ser Gln Leu Ser Thr Arg
    290                 295                 300

Ala Leu Glu Gly Ile Phe Glu Ala Thr His Arg Leu Ile Tyr Gly Ala
305                 310                 315                 320

Lys Asp Asp Ser Gly Gln Arg Tyr Leu Ala Trp Ser Gly His Ser Ala
                325                 330                 335

Arg Val Gly Ala Ala Arg Asp Met Ala Arg Ala Gly Val Ser Ile Pro
            340                 345                 350

Glu Ile Met Gln Ala Gly Gly Trp Thr Asn Val Asn Ile Val Met Asn
        355                 360                 365

Tyr Ile Arg Asn Leu Asp Ser Glu Thr Gly Ala Met Val Arg Leu Leu
    370                 375                 380

Glu Asp Gly Asp
385

## Description

The present invention relates to the regulation of the Cre recombinase by a process using a dissociation/re-association system of the Cre recombinase.

The Cre/LoxP system is used to modify the expression of a selected gene or induce chromosomal rearrangements in a controlled fashion, through the induction of a recombination in the genome of isolated cells or transgenic animals (Sauer, 1994). The system relies on an enzyme of the P1 bacteriophage, Cre recombinase, that recognizes a short (34bp) asymmetric consensus sequence called LoxP. When two such sequences of same orientation are located on a DNA molecule, the enzyme will catalyze the recombination between these two sites and excise the intervening DNA segment. If the two LoxP sequences are located on two different DNA molecules - for example on two chromosomes - Cre will mediate an intermolecular recombination.

In practice, the use of this system to stop the expression of a gene requires the introduction, into the genome, of a LoxP sequence on each side of the gene to be inactivated. When the aim is to activate the expression of the gene, the sequence of this gene have to be preceded by a sequence containing a stop codon flanked by two LoxP sequences. The expression of Cre in the cells will lead, in a second step, to the excision of the sequence between the LoxP sequences and, consequently, to the inactivation (or activation) of the gene.

The *in vivo,* or even the *in vitro,* application of this system requires that the activity of Cre is modulated in a time- and site-specific manner (Metzger and Feil, 1999). Spatial regulation, i.e. the restriction of Cre expression to specific cell types, can be obtained by placing the recombinase under the control of a cell-type specific promoter. Temporal modulation of Cre has been more difficult to achieve. Indeed, a reliable modulation demands both a complete absence of activity in baseline -non-activated- conditions, and a complete excision in the totality of the targeted cells upon activation of Cre. Existing solutions do not simultaneously satisfy these two criteria and the present procedure aims at obtaining a more strict temporal regulation of Cre.

To this day two classes of regulatable Cre expression vectors have been described: steroid-regulatable systems (Brocard et al., 1998; Feil et al., 1996; Kelledonk et al., 1996) and systems based on the regulation of Cre transcription by tetracyclin (St-Onge et al., 1996).

Both above-mentioned systems are relatively leaky, i.e. even in conditions where no Cre activity should be observed because of lack of transcription (tetracyclin-regulated systems) or because of blockade of the biological activity (steroid-regulated systems), there is a non-negligible level of recombination.

Thus, there is a need for a modulatable Cre vector with strict regulation: absence of baseline activity and high level induction.

The aim of the present invention is to provide a process of regulation of Cre recombinase by dissociation/re-association of the recombinase, said process presenting a lower baseline activity and a high level of induction.

The present invention also provides a system that should represent an optimal solution for cases where temporal regulation of Cre is necessary such as induction or inactivation of genes in cell or gene-therapy situations or, in basic research, for conditional transgenesis.

The present invention relates to the use of:
- a first nucleotide sequence encoding a first polypeptide sequence comprising a N-terminal fragment of the P1 bacteriophage Cre recombinase as represented by SEQ ID NO : 2, said N-terminal fragment being linked to a first peptide,
- and of a second nucleotide sequence encoding a second polypeptide sequence comprising a C-terminal fragment of said Cre recombinase, said C-terminal fragment being linked to a second peptide,
said first and second peptides being able to bind together only in the presence of a specific third molecule,
- or of vectors containing said first and second nucleotide sequences,
- or of said first and second polypeptide sequences,
for carrying out a process for regulating the activity of the P1 bacteriophage Cre recombinase as represented by SEQ ID NO : 2, said process being characterized in that in the absence of the third specific molecule in the medium containing said first and second polypeptide sequences, there is no Cre recombinase activity, and in that the addition to said medium of the third specific molecule leads to the formation of a complex between said third molecule and the first and second peptides, and thus to the physical association of said first and second polypeptide sequences in said complex, leading to a Cre recombinase activity.

The Cre recombinase activity can be measured according the experimental section described hereafter.

The present invention also relates to the use of nucleotide sequences encoding said first and second polypeptides sequences, or of vectors containing said nucleotide sequences, as defined above, for regulating the activity of the Cre recombinase in transformed cells containing nucleotide sequences encoding said first and second polypeptides, in particular in the frame of processes of inactivation or activation of the expression of a selected gene, or for inducing chromosomal deletion, or reciprocal chromosomal translocation, or for exchanging one DNA cassette for another, in said transformed cells, or for obtaining cells, and more particularly. mammalian cells, genetically transformed with nucleotide sequences encoding said first and second polypeptides and when appropriate a selected gene, said genetically transformed cells being useful as pharmaceutical compositions in the frame of gene therapy, or for obtaining transgenic animals the genome of which containing said nucleotide sequences and wherein the expression of a selected gene can be activated or inactivated.

The present invention also relates to the use as defined above, characterized in that the N-terminal fragment of the Cre recombinase comprises approximately the 50 to 100 first aminoacids of the Cre recombinase, wherein the 20 first aminoacids can be cancelled, and the C-terminal fragment of the Cre recombinase comprises approximately the 200 to 300 last aminoacids of the Cre recombinase.

The present invention also relates to the use as defined above, characterized in that:
- the N-terminal fragment of the Cre recombinase is chosen among:
   * the fragment delimited in its N-terminal extremity by an aminoacid located between positions 1 to 19 of the Cre recombinase represented by SEQ ID NO : 2, and in its C-terminal extremity by the aminoacid located in position 104, of said Cre recombinase, and more particularly the fragment delimited by the aminoacids located in positions 19 to 104 of the Cre recombinase, and represented by SEQ ID NO : 4,
   * the fragment delimited in its N-terminal extremity by an aminoacid located between positions 1 to 19 of the Cre recombinase represented by SEQ ID NO : 2, and in its C-terminal extremity by the aminoacid located in position 59, of said Cre recombinase, and more particularly the fragment delimited by the aminoacids located in positions 19 to 59 of the Cre recombinase, and represented by SEQ ID NO : 6,
- and the C-terminal fragment of the Cre recombinase is chosen among respectively:
   * the fragment delimited by the aminoacids located in positions 105 or 106 to 343 of the Cre recombinase represented by SEQ ID NO : 2, and more particularly the fragment delimited by the aminoacids located in positions 106 to 343 of the Cre recombinase, and represented by SEQ ID NO : 8,
   * the fragment delimited by the aminoacids located in positions 60 to 343 of the Cre recombinase represented by SEQ ID NO : 2, and represented by SEQ ID NO : 10.

The present invention also relates to the use as defined above, characterized in that:
- the first peptide linked to the N-terminal fragment is the FKBP human protein represented by SEQ ID NO : 12, and the second peptide linked to the C-terminal fragment is the rapamycin binding domain delimited by the aminoacids located in positions 2021 to 2112 or 2113 of the human FRAP protein, if necessary with a T2098L mutation, such as the FRB protein represented by SEQ ID NO : 14, or conversely, the first peptide linked to the N-terminal fragment is said rapamycin binding domain, such as the FRB protein represented by SEQ ID NO : 14, and the second peptide linked to the C-terminal fragment is the FKBP human protein represented by SEQ ID NO : 12,
- the third molecule responsible for the binding of FRB and FKBP is the rapamycin or its analog AP21967 of formula:

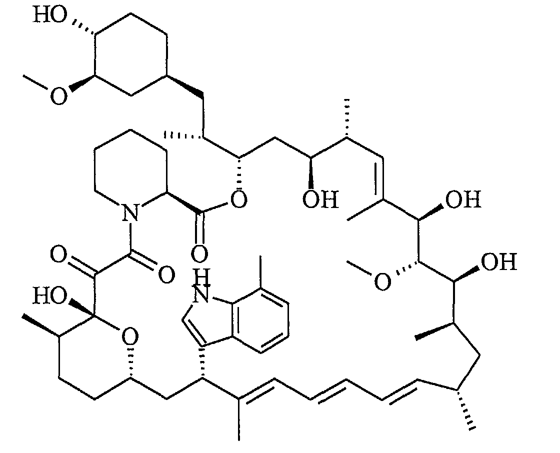

The present invention also relates to the use as defined above, characterized in that the first and second peptides are linked to the N-terminal extremity of the N-terminal and C-terminal fragments of the Cre recombinase, if necessary via a peptide linker of approximately 5 to 30 aminoacids, said first and second peptides being possibly linked to a nuclear localization signal (NLS), itself possibly linked to a transcription improving sequence such as a Kozak sequence.

The present invention also relates to the use as defined above, characterized in that the peptide linker is chosen among SEQ ID NO : 16 to SEQ ID NO : 38, the nuclear localization signal is the sequence SEQ ID NO : 40, and the Kozak sequence is the sequence SEQ ID NO : 42.

The present invention also relates to the use as defined above, characterized in that:
- the first polypeptide sequence is chosen among the following sequences:
   * the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FKBP sequence, the FKBP sequence represented by SEQ ID NO : 12, a linker chosen among SEQ ID NO : 16 to SEQ ID NO : 38, and the fragment delimited by the aminoacids located in positions 19 to 104 of the Cre recombinase, and represented by SEQ ID NO : 4, said first polypeptide sequence corresponding to SEQ ID NO : 44,
   * the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FKBP sequence, the FKBP sequence represented by SEQ ID NO : 12, a linker chosen among SEQ ID NO : 16 to SEQ ID NO : 38, and the fragment delimited by the aminoacids located in positions 19 to 59 of the Cre recombinase, and represented by SEQ ID NO : 6, said first polypeptide sequence corresponding to SEQ ID NO : 46,
- and the second polypeptide sequence is chosen among the following sequences:
   * the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FRB sequence, the FRB sequence represented by SEQ ID NO : 14, a linker chosen among SEQ ID NO : 16 to SEQ ID NO : 38, and the fragment delimited by the aminoacids located in positions 106 to 343 of the Cre recombinase, and represented by SEQ ID NO : 8, said first polypeptide sequence corresponding to SEQ ID NO : 48,
   * the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FRB sequence, the FRB sequence represented by SEQ ID NO : 14, a linker chosen among SEQ ID NO : 16 to SEQ ID NO : 38, and the fragment delimited by the aminoacids located in positions 60 to 343 of the Cre recombinase, and represented by SEQ ID NO : 10, said first polypeptide sequence corresponding to SEQ ID NO : 50.

The present invention also relates to polypeptides sequences corresponding to N-terminal fragments of the P1 bacteriophage Gre recombinase as represented by SEQ ID NO : 2, comprising approximately the 50 to 100 first aminoacids of the Cre recombinase, wherein the 20 first aminoacids can be cancelled.

According to an advantageous embodiment, the present invention also relates to the above-mentioned polypeptides sequences chosen among:
* the fragment delimited in its N-terminal extremity by an aminoacid located between positions 1 to 19 of the Cre recombinase represented by SEQ ID NO : 2, and in its C-terminal extremity by the aminoacid located in position 104, of said Cre recombinase, and more particularly the fragment delimited by the aminoacids located in positions 19 to 104 of the Cre recombinase, and represented by SEQ ID NO : 4,
* the fragment delimited in its N-terminal extremity by an aminoacid located between positions 1 to 19 of the Cre recombinase represented by SEQ ID NO : 2, and in its C-terminal extremity by the aminoacid located in position 59, of said Cre recombinase, and more particularly the fragment delimited by the aminoacids located in positions 19 to 59 of the Cre recombinase, and represented by SEQ ID NO:6.

The present invention also relates to polypeptides sequences corresponding to C-terminal fragment of the P1 bacteriophage Cre recombinase as represented by SEQ ID NO : 2, comprising approximately the 200 to 300 last aminoacids of the Cre recombinase.

According to an advantageous embodiment, the above-mentioned polypeptides sequences of the invention are chosen among:
* the fragment delimited by the aminoacids located in positions 105 or 106 to 343 of the Cre recombinase represented by SEQ ID NO : 2, and more particularly the fragment delimited by the aminoacids located in positions 106 to 343 of the Cre recombinase, and represented by SEQ ID NO : 8,
* the fragment delimited by the aminoacids located in positions 60 to 343 of the Cre recombinase represented by SEQ ID NO : 2, and represented by SEQ ID NO : 10.

The present invention also relates to the above-mentioned polypeptides sequences, linked to the FKBP human protein represented by SEQ ID NO : 12, or to the rapamycin binding domain delimited by the aminoacids located in positions 2021 to 2112 or 2113 of the human FRAP protein, if necessary with a T2098L mutation, such as the FRB protein represented by SEQ ID NO : 14.

The present invention also relates to polypeptides sequences such as defined previously, characterized in that the FKBP or FRB proteins are linked to the N-terminal extremity of the N-terminal and C-terminal fragments of the Cre recombinase, if necessary via a peptide linker of approximately 5 to 30 aminoacids, said FKBP or FRB proteins being possibly linked to a nuclear localization signal (NLS), itself possibly linked to a transcription improving sequence such as a Kozak sequence.

According to an advantageous embodiment, the polypeptides sequences according to the invention are characterized in that the peptide linker is chosen among SEQ ID NO : 16 to SEQ ID NO : 38, the nuclear localization signal is the sequence SEQ ID NO : 40, and the Kozak sequence is the sequence SEQ ID NO : 42.

According to an advantageous embodiment, the polypeptides sequences, such as mentioned previously, are chosen among the following sequences:
* the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FKBP sequence, the FKBP sequence represented by SEQ ID NO : 12, a linker chosen among SEQ ID NO : 16 to SEQ ID NO : 38, and the fragment delimited by the aminoacids located in positions 19 to .104 of the Cre recombinase, and represented by SEQ ID NO : 4, said first polypeptide sequence corresponding to SEQ ID NO : 44,
* the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FKBP sequence, the FKBP sequence represented by SEQ ID NO : 12, a linker chosen among SEQ ID NO : 16 to SEQ ID NO : 38, and the fragment delimited by the aminoacids located in positions 19 to 59 of the Cre recombinase, and represented by SEQ ID NO : 6, said first polypeptide sequence corresponding to SEQ ID NO : 46,

- and the second polypeptide sequence is chosen among the following sequences:
   * the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FRB sequence, the FRB sequence represented by SEQ ID NO : 14, a linker chosen among SEQ ID NO : 16 to SEQ ID NO : 38, and the fragment delimited by the aminoacids located in positions 106 to 343 of the Cre recombinase, and represented by SEQ ID NO : 8, said first polypeptide sequence corresponding to SEQ ID NO : 48,
   * the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FRB sequence, the FRB sequence represented by SEQ ID NO : 14, a linker chosen among SEQ ID NO : 16 to SEQ ID NO : 38, and the fragment delimited by the aminoacids located in positions 60 to 343 of the Cre recombinase, and represented by SEQ ID NO : 10, said first polypeptide sequence corresponding to SEQ ID NO : 50.

The present invention also relates to nucleotide sequences encoding a polypeptide such as defined previously.

According to an advantageous embodiment, the nucleotide sequences according to the invention are chosen among SEQ ID NO : 43, 45, 47, and 49, and coding respectively for the polypeptides sequences SEQ ID NO : 44, 46, 48, and 50.

The present invention also relates to vectors, such as plasmids, containing a nucleotide sequence such as defined above.

The present invention also relates to cells transformed with a vector such as defined previously.

The present invention also relates to a process for regulating the activity of the P1 bacteriophage Cre recombinase as represented by SEQ ID NO : 2 in cells characterized in that it comprises:
- the transformation of cells with two vectors, one vector containing a first nucleotide sequence encoding a first polypeptide sequence as defined above, and the other vector containing a second sequence nucleotide encoding a second polypeptide sequence as defined above, or the transformation of cells with one vector containing said first and second nucleotide sequences,
- the addition to said cell of the third molecule as defined above liable to form a complex with the first and second peptides comprised in the first and second polypeptide sequences mentioned above, thus leading to the physical association of the said first and second polypeptide sequences, and to a Cre recombinase activity.

The invention also relates to a process for the *in vitro* inactivation of a selected gene characterized in that it comprises:
- the transformation of cells with two vectors, one vector containing a first sequence encoding a first polypeptide sequence as defined above, and the other vector containing a second sequence encoding a second polypeptide sequence as defined above, or the transformation of cells with one vector containing said first and second nucleotide sequences, said cells being also transformed in such way that said selected gene, or a functionally essential part of said gene, is flanked upstream and downstream with two sequences encoding LoxP, or equivalent sequences (such as Lox511 described in Langer et al, 2002, Nucl. Acid Res., 30 : 3067-3077), in parallel orientation,
- the addition to said transformed cells of the said specific third molecule leading to a Cre recombinase activity and to the inactivation of said selected gene.

The invention also relates to a process for inducing chromosomal deletion characterized in that it comprises:
- the transformation of cells with two vectors, one vector containing a first sequence encoding a first polypeptide sequence as defined above, and the other vector containing a second sequence encoding a second polypeptide sequence as defined above, or the transformation of cells with one vector containing said first and second nucleotide sequences, said cells being also transformed in such way that two sequences encoding LoxP, or equivalent sequences, in parallel orientation are introduced into a selected chromosome, delimitating the part to be excised from said chromosome,
- the addition to said transformed cells of the specific third molecule defined above, leading to a Cre recombinase activity and to the deletion of the LoxP-flanked chromosomal region.

The invention also relates to a process for inducing reciprocal chromosomal translocation characterized in that it comprises:
- the transformation of cells with two vectors, one vector containing a first sequence encoding a first polypeptide sequence as defined above, and the other vector containing a second sequence encoding a second polypeptide sequence as defined above, or the transformation of cells with one vector containing said first and second nucleotide sequences, said cells being also transformed in such way that one sequence encoding LoxP, or equivalent sequence, is introduced into each of two selected chromosomes, at the sites at which the translocation should take place,
- the addition to said transformed cells of the specific third molecule defined above, leading to a Cre recombinase activity and to interchromosomal recombination.

The invention also relates to a process for the *in vitro* activation of a selected gene characterized in that it comprises:
- the transformation of cells with two vectors, one vector containing a first sequence encoding a first polypeptide sequence as defined above, and the other vector containing a second sequence encoding a second polypeptide sequence as defined above, or the transformation of cells with one vector containing said first and second nucleotide sequences, said cells being also transformed in such way that a sequence containing a stop codon flanked with two sequences encoding LoxP, or equivalent sequences, of parallel orientation is inserted upstream said selected gene, in front of its start codon,
- the addition to said transformed cells of the specific third molecule defined above, leading to a Cre recombinase activity and to excision of said LoxP-flanked sequences and the transcription of said selected gene.

The invention also relates to a process for exchanging one DNA cassette for another by recombinase-mediated cassette exchange characterized in that it comprises:
- the transformation of cells with two vectors, one vector containing a first sequence encoding a first polypeptide sequence as defined above, and the other vector containing a second sequence encoding a second polypeptide sequence as defined above, or the transformation of cells with one vector containing said first and second nucleotide sequences, said cells being also transformed in such way that a target sequence is flanked by two sequences encoding LoxP, or equivalent sequences, containing complementary mutations within their palindromic arms, and one of them containing also a mutation within the spacer region,
- the introduction into said transformed cells of a targeting vector containing a cassette flanked by same variant of sequences encoding LoxP,
- the addition to said transformed cells of the specific third molecule defined above, leading to a Cre recombinase activity and to the exchange of the target sequence with the cassette contained in the said targeting vector.

The invention also relates to a process for the inactivation of a selected gene in transgenic animals characterized in that it comprises:
- the introduction into a transgenic line with two constructs, one construct containing a first sequence encoding a first polypeptide sequence as defined above, and the other construct containing a second sequence encoding a second polypeptide sequence as defined above, or the transformation of said transgenic line with one vector containing said first and second nucleotide sequences, said transgenic line being homozygous for a modified allele of a selected gene that have been transformed in such way that said gene or a functionally essential part of said gene is flanked upstream and downstream with two sequences encoding LoxP, or equivalent sequences, in parallel orientation,
- the treatment of transgenic animals obtained from said transgenic line with the specific third molecule defined above, leading to a Cre recombinase activity and to inactivation of said selected gene.

### LEGENDS TO THE FIGURES

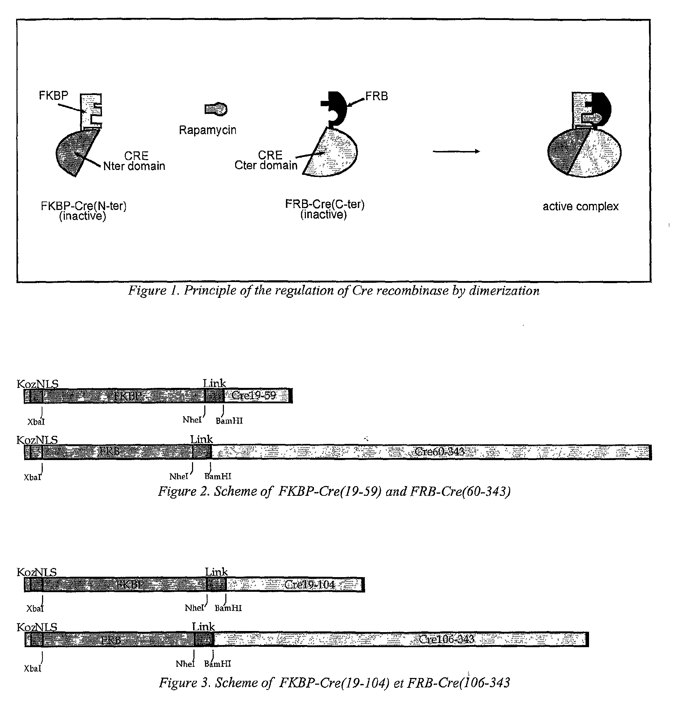

Figure 1 describes the principle of the regulation of Cre recombinase by the system of dissociation/re-association of the Cre recombinase according to the invention.

Figure 2 represents the constructions FKBP-Cre(19-59) and FRB-Cre(60-343). Koz corresponds to a Kozak sequence; NLS corresponds to a nuclear localization signal; FKBP corresponds to FKBP12 ("FK506 binding protein"); FRB corresponds to the ligand recognition domain of FRAP ("FKBP and rapamycin associated protein"); "Link" corresponds to a peptide linker; "Cre19-59" corresponds to the fragment delimited by the aminoacids located in positions 19 to 59 of the Cre recombinase; "Cre60-343" corresponds to the fragment delimited by the aminoacids located in positions 60 to 343 of the Cre recombinase.

Figure 3 represents the constructions FKBP-Cre(19-104) and FRB-Cre(106-343). Koz corresponds to a Kozak sequence; NLS corresponds to a nuclear localization signal; FKBP corresponds to FKBP12 ("FK506 binding protein"); FRB corresponds to the ligand recognition domain of FRAP ("FKBP and rapamycin associated protein"); "Link" corresponds to a peptide linker; "Cre19-104" corresponds to the fragment delimited by the aminoacids located in positions 19 to 104 of the Cre recombinase; "Cre106-343" corresponds to the fragment delimited by the aminoacids located in positions 106 to 343 of the Cre recombinase.

### EXPERIMENTAL

The regulation of Cre recombinase relies on the dimerization (association), mediated by a specific drug, of two complementing moieties (an N-terminal and a C-terminal domain) of the enzyme. To obtain this dimerization, each of the two moieties are fused to a protein to which the drug can bind specifically and with a high affinity (Spencer, 1996; Rivera et al., 1996). Thus, the N-terminal domain is fused to FKBP (FKBP12, for "FK506 binding protein"; Harding et al., 1989; Sikierka et al., 1989), while the C-terminal domain is fused to the ligand recognition domain of FRAP ("FKBP and rapamycin associated protein") called FRB (Chen et al., 1995). The version of FRB used has a T2098L point mutation that enables it to bind analogs of rapamycin devoid of binding to wild type FRAP. Neither of the two fusion proteins possesses any enzymatic activity of its own, even if colocalized in the same cell. On the other hand their association by rapamycin, that binds with a nanomolar affinity to FKBP and to FRB, leads to a reconstitution of the enzymatic activity (Fig. 1). The use of two different proteins (FRB and FKBP) and of rapamycin precludes the formation of (inactive) homodimers that would compete with the formation of the active complex.

The method is based on the use of two expression vectors: one to express the fusion protein FKBP-Cre(N-ter) and a second to express the fusion protein FRB-Cre(C-ter). FKBP and FRB are linked to the respective Cre moiety by a short peptide ("Link"). This linker peptide is flanked by unique restriction sites (NheI and BamH1) allowing its exchange with different peptides to test the influence of different linker conformations on the performance of the system. For each of these fusion proteins two versions have been made, depending on the site at which Cre has been cut: between amino acids 59 and 60 or between amino acids 104 and 106. Thus two pairs of fusion proteins have been obtained: FKBP-Cre(19-59) and FRB-Cre(60-343) on one hand, FKBP-Cre(19-104) and FRB-Cre(106-343) on the other hand. In these fusion proteins FKBP was fused with the N-terminal half of the N-terminal moiety of Cre, at amino acid 19 of Cre (i.e. the first 18 N-terminal residues of Cre have not been included in the fusion construct), while FRB was fused with the amino acid 60 or 106 of Cre, i.e. at the N-terminal end of the C-terminal half of Cre. The sites of the cuts, the linkers and the site of fusions have been designed on the basis of published structures of Cre (Guo et al., 1997) and of the FKBP-rapamycin-FRB complex (Liang et al., 1999). The fragments coding for the fusion proteins have been obtained by PCR and controlled by sequencing. Each fusion protein begins with a nuclear localization signal ("NLS") and the coding sequence is preceeded by a Kozak sequence ("Koz") to improve its transcription (see Figs. 2 and 3).

Plasmid pairs are co-introduced in the target cells by a standard transfection procedure. Cre activity is induced by adding 1-10 nM rapamycin to the cells, 12-15 h after the transfection. The cells are fixed 48 h after transfection and LacZ activity revealed using a standard histochemical procedure with X-gal as substrate.

### MATERIALS AND METHODS

### Elements of the system and overview of the constructs

Cre has been obtained from iCre, an improved version of Cre, with human codon-usage preferences, minimized CpG content and elimination of putative cryptic splice sites (Shimshek et al., 2002). Note that in the final constructs the first 18 amino acids of Cre have not been included (see also below).

FKBP12 (FKBP, for "FK506 binding protein") is the full-length human protein that is the initial intracellular target for the immunosuppressive drugs FK506 and rapamycin (Harding et al., 1989; Sikierka et al., 1989). FRB ("FKBP12 Rapamycin Binding domain") corresponds to the rapamycin-binding domain of FRAP ("FKBP and rapamycin associated protein", also called RAFT, RAPT, mTOR; Chen et al., 1995; Raught et al., 2001) i.e. aa 2021-2113 of human FRAP, with a T2098L point mutation that enables it to bind analogs of rapamycin devoid of binding to wild type FRAP. Note that in our constructs Lys(2113) has not been included. Both FKBP and FRB have been obtained from ARIAD Pharmaceuticals (Cambridge, MA).

The method is based on the use of two expression vectors: one to express the fusion protein FKBP-Cre(N-ter) and a second to express the fusion protein FRB-Cre(C-ter). FKBP and FRB are linked to the respective Cre moieties by a short peptide linker (Lk). For each of these fusion proteins two versions have been made, depending on the site at which Cre has been cut: between amino acids 59 and 60 or between amino acids 104 and 106. Thus two pairs of fusion proteins have been obtained: FKBP-Lk-Cre(19-59) and FRB-Lk-Cre(60-343) on one hand, FKBP-Lk-Cre(19-104) and FRB-Lk-Cre(106-343) on the other hand. In these fusion proteins FKBP was fused with the N-terminal half of the N-terminal moiety of Cre, at amino acid 19 of Cre, while FRB was fused with the amino acid 60 or 106 of Cre, i.e. at the N-terminal end of the C-terminal half of Cre. The sites of the cuts, the linkers and the site of fusions have been designed on the basis of published structures of Cre (Guo et al., 1997) and of the FKBP-rapamycin-FRB complex (Liang et al., 1999) using the Insight modelling package (Accelrys Company). Note that the published Cre structure does not include the first 18 N-terminal amino acids and, to be in conformity with the known structure when modelling the projected constructs, we have left out this portion in our constructs. Each fusion protein begins with a nuclear localization signal (NLS) and the coding sequence is preceded by a Kozak sequence (Koz) to improve its transcription.

### Vector construction

Sequences coding for the fusion proteins have been constructed by PCR cloning (Ausabel et al., 2002) starting from plasmids pNLS-iCre for Cre, pCF1E for FKBP and pCGNN-FRB(H1) for FRB (both obtained from ARIAD Pharmaceuticals, Cambridge, MA) and cloned into pcDNA3.1 expression vector (Invitrogen, Groeningen, Holland). First the vectors with linker # 1 (Lk1) have been synthesized. In the resulting constructs the linker was flanked by unique restriction sites (Nhe1 and BamH1), allowing the simple replacement of Lkl by the other linkers to be tested by digesting the vector with these enzymes and ligating in custom made oligonucleotides that have been synthesized so as to possess ends that were cohesive with the digested vector ends. All the constructs have been sequenced to confirm their conformity with the predicted sequence.

### pcDNA-KozNLS-FKBP-Lk1-Cre(19-59) and pcDNA-KozNLS-FKBP-Lk1-Cre(19-104)

FKBP has been amplified by PCR using Pfu (Promega) from pCF1E using primers 1 and 2 (annealing temperature: 5x50°C followed by 20x72°C, elongation time: 1 min). Cre(19-59) has been amplified from pNLS-iCre using primers 3 and 4 (annealing temperature: 5x55°C followed by 20x61°C, elongation time: 35 sec), while Cre(19-104) has been amplified from the same plasmid using primers 3 and 5 (annealing temperature: 25x55°C, elongation time 35 sec). Note that primers 2 and 3 have a complementary sequence that together form the sequence for Lk1. The amplified fragments were cleaned, to get rid of the primers, and the fragment amplified from FKBP was mixed with the one corresponding to Cre(19-59) or Cre(19-104). A second round of PCR was performed using primers 1 and 4 (to create FKBP-Lk1-Cre(19-59); annealing temperature: 25x58°C, elongation time: 80 sec) or 1 and 5 (to create FKBP-Lk1-Cre(19-104); annealing temperature: 25x58°C, elongation time: 80 sec).

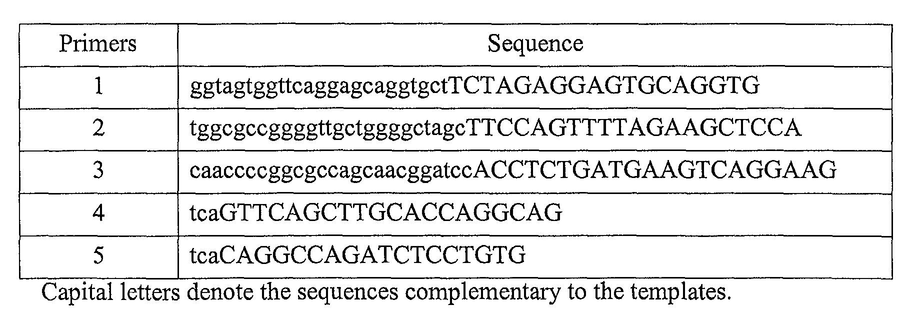

The final amplification products were blunt-ligated into pBS II SK(+) that was cut by BamH1 and blunted, were subsequently excised using XbaI and ligated into a modified pBS II SK(+) that contains a KozNLS sequence terminated by an XbaI site. The resulting KozNLS-FKBP-Lk1-Cre(19-59) and KozNLS-FKBP-Lk1-Cre(19-104) fragments were excised using EcoR1 and NotI and ligated into pcDNA3.1 from which the NheI and BamH1 sites have been removed beforehand.

### pcDNA-KozNLS-FRB-Lk1-Cre(60-343) and pcDNA-KozNLS-FRB-Lk1-Cre(106-343)

FRB has been amplified by PCR using Pfu (Promega) from pCGNN-FRB(H1) using primers 6 and 7 (annealing temperature: 5x50°C followed by 20x68°C, elongation time: 45 sec). Cre(60-343) has been amplified from pNLS-iCre using primers 8 and 9 (annealing temperature: 5x56°C followed by 20x65°C, elongation time: 110 sec), while Cre(106-343) has been amplified from the same plasmid using primers 10 and 9 (annealing temperature: 5x56°C followed by 20x65°C, elongation time 110 sec). Note that primers 7 and 8 as well as primers 7 and 10 have a complementary sequence that together form the sequence for Lk1. The amplified fragments were partially cleaned, to get rid of the primers, and the fragment amplified from FRB was mixed with the one corresponding to Cre(60-343) or Cre(106-343). A second round of PCR was performed using primers 6 and 9 (annealing temperature: 5x59°C followed by 20x65°C, elongation time: 145 sec).

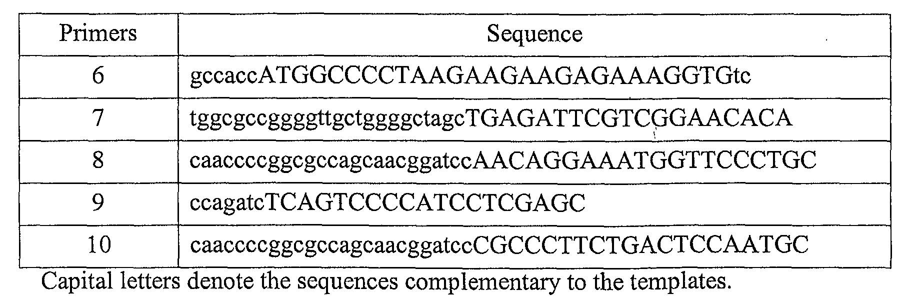

The final amplification products, corresponding to KozNLS-FRB-Lkl-Cre(60-343) and KozNLS-FRB-Lk1-Cre(106-343) were directly ligated into the NotI site of pcDNA3.1 from which the NheI and BamH1 sites have been removed beforehand.

### Cell transfection

Rat2 fibroblasts were propagated in standard culture medium (DMEM containing 10% fetal calf serum, 2 mM glutamine and penicillin/streptomycin) at 37°C in 95% air-5% CO₂ atmosphere. Cells were infected with a self-inactivating retroviral backbone (pSIR; Clontech, Palo Alto, CA) containing the CALNLZ cassette (Kanegae et al., 1995). Infected cells were selectioned with G418 (500 µg/ml) and resistant clones isolated using cloning rings. The resulting Rat2/pSIR-CALNLZ line was used as an indicator line to test Cre recombinase activity. Given the characteristics of this construct, the cells will express the LacZ gene only if a Cre-mediated recombination occurs in the cells.

For transfection, Rat2/pSIR-CALNLZ cells were plated into 24well culture plates (3.5 × 10⁴ cells/well). Six to eighteen hours later, cells were transfected, using polyethylenimine (PEI; Aldrich, St. Louis, MO) with the expression plasmids expressing the constructs to be tested. For transfection of one well, 1 µg DNA in 36 µl 150 mM NaCl was mixed with 14 µl 2 mM PEI. The resulting mixture was deposited, after 30 minutes, onto the cells with 400 µl culture medium and the plates put back to the incubator. The medium was changed 12-15 hours later, 50 ng/ml rapamycin (final concentration; Sigma, St. Louis, MO) was added where needed. Cells were fixed for 5 minutes 48 hours after transfection using a mixture of 2% paraformaldehyde and 0.4% glutaraldehyde and LacZ activity was revealed using X-gal as substrate. The number of blue cells per well was counted under an inverted microscope and the count was taken as the measure of Cre recombinase activity. Experiments were repeated at least 5 times.

### REFERENCES

Ausabel, F.M., Brent, R., Kingston, R.E., Moore, D.D., Seidman, J.G., Smith, J.A. and Struhl, K. (Eds.) Current Protocols in Molecular Biology. John Wiley, New York, 2002.
Brocard, J., Feil, R., Chambon, P. et Metzger, D. (1998) A chimeric Cre recombinase inducible by synthetic, but not by natural ligands of the glucocorticoid receptor. *Nucl. Acid Res.,* **26:** 4086-4090.
Chen, J., Zheng, X.F., Brown, E.J. and Schreiber, S.L. (1995) Identification of an 11 kDa FKBP12-rapamycin-binding domain within the 289 kDa FKBP12-rapamycin-accociated protein and characterization of a critical serine residue. *Proc. Natl. Acad. Sci. U.S.A*., **92:** 4947-51.
Feil, R., Brocard, J., Mascrez, B., leMeur, M. et Chambon, P. (1996) Ligand-activated site-specific recombination in mice. *Proc. Natl. Acad. Sci. U.S.A*., **93**: 10887-10890.
Guo, F., Gopaul, D.N. and van Duyne, G.D. (1997) Structure of Cre recombinase complexed with DNA in a site-specific recombination synapse. *Nature,* **389:** 40-46.
Harding, M.W., Galat, A., Uehling, D.E. and Schreiber, S.L. (1989) A receptor for the immunosuppressant FK506 is a cis-trans peptidyl-prolyl isomerase. Nature, 341: 758-60.
Kanegae, Y., Lee, G., Sato, Y., Tanaka, M., Naki, M., Sakaaki, T., Sugano, S. and Saito, I. (1995) Efficient gene activation in mammalian cells by using recombinant adenovirus expressing site-specific Cre recombinase. *Nucl. Acid Res.,* **23:** 3816-3821.
Kelledonk, C., Tronche, F., Monaghan, A.-P., Angrand, P.-O., Stewart, F. et Schütz, G. (1996) Regulation of Cre recombinase activity by the synthetic steroid RU 486. *Nucl. Acid Res*., **24:** 1404-1411.
Liang, J., Choi, J. and Clardy, J. (1999) Refined structure of the FKBP12-rapamycin-FRB ternary complex at 2.2 Å resolution. *Acta Cryst*., **D55,** 736-744.
Metzger, D. et Feil, R. (1999) Engineering the mouse genome by site-specific recombination. *Curr. Opin. Biotechnol*., **10:** 470-476.
Raught, B., Gingras, A.-C. and Sonenberg, N. (2001) The target of rapamycin (TOR) proteins. *Proc. Natl. Acad. Sci. U.S.A*., **98**: 7037-44.
Rivera, V.M., Clackson, T., Natesan, S., Pollock, R., Amara, J.F., Keenan, T., Magari, S.R., Phillips, T., Courage, N.L., Cerasoli, F., Jr., Holt, D.A. and Gilman, M. (1996) A humanized system for pharmacologic control of gene expression. *Nature Med*., **2**: 1028-32.
Sauer, B. (1994) Site-specific recombination: development and applications. *Curr. Opin. Biotechnol*., **5**: 521-527.
Shimshek, D.R., Kim, J., Hübner, Spergel, D.J., Buchholz, F., Casanova, E., Stewart, A.F., Seeburg, P.H. and Sprengel, R. (2002) Codon-improved Cre recombinase (iCre) expression in the mouse. *Genesis,* **32:** 19-26.
Sikierka, J.J., Hung, H.Y., Poe, M., Lin, C.S. and Sigal, N.S. (1989) A cytosolic binding protein for the immunosuppressant FK506 has peptidyl-prolyl isomerase activity but is distinct from cyclophylin. *Nature,* **341:** 755-757
Spencer, D.M. (1996) Creating conditional mutations in mammals. *Trends Genet.,* **12**: 181187
St-Onge, L., Furth, P.A. et Gruss, P. (1996) Temporal control of the Cre recombinase in transgenic mice by a tetracycline-responsive promoter. *Nucl. Acid Res.,* **24**: 3875-3877.

## Claims

1. Use of:
- a first nucleotide sequence encoding a first polypeptide sequence comprising a N-terminal fragment of the P1 bacteriophage Cre recombinase as represented by SEQ ID NO : 2, said N-terminal fragment being linked to a first peptide,
- and of a second nucleotide sequence encoding a second polypeptide sequence comprising a C-terminal fragment of said Cre recombinase, said C-terminal fragment being linked to a second peptide,
said first and second peptides being able to bind together only in the presence of a specific third molecule,
- or of vectors containing said first and second nucleotide sequences,
- or of said first and second polypeptide sequences,
for carrying out a process for regulating the activity of the P1 bacteriophage Cre recombinase as represented by SEQ ID NO : 2, said process being **characterized in that** in the absence of the third specific molecule in the medium containing said first and second polypeptide sequences, there is no Cre recombinase activity, and **in that** the addition to said medium of the third specific molecule leads to the formation of a complex between said third molecule and the first and second peptides, and thus to the physical association of said first and second polypeptide sequences in said complex, leading to a Cre recombinase activity resulting from the association of said first and second polypeptide sequences.

2. Use of nucleotide sequences encoding said first and second polypeptides sequences, or of vectors containing said nucleotide sequences, according to claim 1, for regulating the activity of the Cre recombinase in transformed cells containing nucleotide sequences encoding said first and second polypeptides, in particular in the frame of processes of inactivation or activation of the expression of a selected gene, or for inducing chromosomal deletion, or reciprocal chromosomal translocation, or for exchanging one DNA cassette for another, in said transformed cells, or for obtaining cells transformed with nucleotide sequences encoding said first and second polypeptides that can be used in the frame of gene therapy, or for obtaining transgenic animals the genome of which containing said nucleotide sequences and wherein the expression of a selected gene can be activated or inactivated.

3. Use according to claim 1 or 2, **characterized in that** the N-terminal fragment of the Cre recombinase comprises approximately the 50 to 100 first aminoacids of the Cre recombinase, wherein the 20 first aminoacids can be cancelled, and the C-terminal fragment of the Cre recombinase comprises approximately the 200 to 300 last aminoacids of the Cre recombinase.

4. Use according to any of claims 1 to 3, **characterized in that**:
- the N-terminal fragment of the Cre recombinase is chosen among:
* the fragment delimited in its N-terminal extremity by an aminoacid located between positions 1 to 19 of the Cre recombinase represented by SEQ ID NO : 2, and in its C-terminal extremity by the aminoacid located in position 104, of said Cre recombinase, and more particularly the fragment delimited by the aminoacids located in positions 19 to 104 of the Cre recombinase, and represented by SEQ ID NO : 4,
* the fragment delimited in its N-terminal extremity by an aminoacid located between positions 1 to 19 of the Cre recombinase represented by SEQ ID NO : 2, and in its C-terminal extremity by the aminoacid located in position 59, of said Cre recombinase, and more particularly the fragment delimited by the aminoacids located in positions 19 to 59 of the Cre recombinase, and represented by SEQ ID NO : 6,
- and the C-terminal fragment of the Cre recombinase is chosen among respectively:
* the fragment delimited by the aminoacids located in positions 105 or 106 to 343 of the Cre recombinase represented by SEQ ID NO : 2, and more particularly the fragment delimited by the aminoacids located in positions 106 to 343 of the Cre recombinase, and represented by SEQ ID NO : 8,
* the fragment delimited by the aminoacids located in positions 60 to 343 of the Cre recombinase represented by SEQ ID NO : 2, and represented by SEQ ID NO : 10.

5. Use according to any of claims 1 to 4, **characterized in that**:
- the first peptide linked to the N-terminal fragment is the FKBP human protein represented by SEQ ID NO : 12, and the second peptide linked to the C-terminal fragment is the rapamycin binding domain delimited by the aminoacids located in positions 2021 to 2112 or 2113 of the human FRAP protein, if necessary with a T2098L mutation, such as the FRB protein represented by SEQ ID NO : 14, or conversely, the first peptide linked to the N-terminal fragment is said rapamycin binding domain, such as the FRB protein represented by SEQ ID NO : 14, and the second peptide linked to the C-terminal fragment is the FKBP human protein represented by SEQ ID NO : 12,
- the third molecule responsible for the binding of FRB and FKBP is the rapamycin or its analog AP21967.

6. Use according to any of claims 1 to 5, **characterized in that** the first and second peptides are linked to the N-terminal extremity of the N-terminal and C-terminal fragments of the Cre recombinase, if necessary via a peptide linker of approximately 5 to 30 aminoacids, said first and second peptides being possibly linked to a nuclear localization signal (NLS), itself possibly linked to a transcription improving sequence such as a Kozak sequence.

7. Use according to any of claims 1 to 6, **characterized in that** the peptide linker is chosen among SEQ ID NO : 16 to SEQ ID NO : 38, the nuclear localization signal is the sequence SEQ ID NO : 40, and the Kozak sequence is the sequence SEQ ID NO : 42.

8. Use according to any of claims 1 to 7, **characterized in that**:
- the first polypeptide sequence is chosen among the following sequences:
* the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FKBP sequence, the FKBP sequence represented by SEQ ID NO : 12, a linker chosen among SEQ ID NO : 16 to SEQ ID NO : 38, and the fragment delimited by the aminoacids located in positions 19 to 104 of the Cre recombinase, and represented by SEQ ID NO : 4, said first polypeptide sequence corresponding to SEQ ID NO : 44,
* the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FKBP sequence, the FKBP sequence represented by SEQ ID NO : 12, a linker chosen among SEQ ID NO : 16 to SEQ ID NO : 38, and the fragment delimited by the aminoacids located in positions 19 to 59 of the Cre recombinase, and represented by SEQ ID NO : 6, said first polypeptide sequence corresponding to SEQ ID NO : 46,
- and the second polypeptide sequence is chosen among the following sequences:
* the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FRB sequence, the FRB sequence represented by SEQ ID NO : 14, a linker chosen among SEQ ID NO : 16 to SEQ ID NO : 38, and the fragment delimited by the aminoacids located in positions 106 to 343 of the Cre recombinase, and represented by SEQ ID NO : 8, said first polypeptide sequence corresponding to SEQ ID NO : 48,
* the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FRB sequence, the FRB sequence represented by SEQ ID NO : 14, a linker chosen among SEQ ID NO : 16 to SEQ ID NO : 38, and the fragment delimited by the aminoacids located in positions 60 to 343 of the Cre recombinase, and represented by SEQ ID NO : 10, said first polypeptide sequence corresponding to SEQ ID NO : 50.

9. Polypeptides sequences corresponding to N-terminal fragments of the P1 bacteriophage Cre recombinase as represented by SEQ ID NO: 2, comprising approximately the 50 to 100 first aminoacids of the Cre recombinase, wherein the 20 first aminoacids can be cancelled.

10. Polypeptides sequences according to claim 9 chosen among:
* the fragment delimited in its N-terminal extremity by an aminoacid located between positions 1 to 19 of the Cre recombinase represented by SEQ ID NO : 2, and in its C-terminal extremity by the aminoacid located in position 104, of said Cre recombinase, and more particularly the fragment delimited by the aminoacids located in positions 19 to 104 of the Cre recombinase, and represented by SEQ ID NO : 4,
* the fragment delimited in its N-terminal extremity by an aminoacid located between positions 1 to 19 of the Cre recombinase represented by SEQ ID NO : 2, and in its C-terminal extremity by the aminoacid located in position 59, of said Cre recombinase, and more particularly the fragment delimited by the aminoacids located in positions 19 to 59 of the Cre recombinase, and represented by SEQ ID NO:6.

11. Polypeptides sequences corresponding to C-terminal fragment of the P1 bacteriophage Cre recombinase as represented by SEQ ID NO: 2, comprising approximately the 200 to 300 last aminoacids of the Cre recombinase.

12. Polypeptides sequences according to claim 11 chosen among :
* the fragment delimited by the aminoacids located in positions 105 or 106 to 343 of the Cre recombinase represented by SEQ ID NO : 2, and more particularly the fragment delimited by the aminoacids located in positions 106 to 343 of the Cre recombinase, and represented by SEQ ID NO : 8,
* the fragment delimited by the aminoacids located in positions 60 to 343 of the Cre recombinase represented by SEQ ID NO : 2, and represented by SEQ ID NO : 10.

13. Polypeptides sequences according to any of claims 9 to 12, linked to the FKBP human protein represented by SEQ ID NO : 12, or to the rapamycin binding domain delimited by the aminoacids located in positions 2021 to 2112 or 2113 of the human FRAP protein, if necessary with a T2098L mutation, such as the FRB protein represented by SEQ ID NO: 14.

14. Polypeptides sequences according to any of claims 9 to 13, **characterized in that** the FKBP or FRB proteins are linked to the N-terminal extremity of the N-terminal and C-terminal fragments of the Cre recombinase, if necessary via a peptide linker of approximately 5 to 30 aminoacids, said FKBP or FRB proteins being possibly linked to a nuclear localization signal (NLS), itself possibly linked to a transcription improving sequence such as a Kozak sequence.

15. Polypeptides sequences according to any of claims 9 to 14, **characterized in that** the peptide linker is chosen among SEQ ID NO : 16 to SEQ ID NO : 38, the nuclear localization signal is the sequence SEQ ID NO : 40, and the Kozak sequence is the sequence SEQ ID NO : 42.

16. Polypeptides sequences according to any of claims 9 to 15, chosen among the following sequences:
* the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FKBP sequence, the FKBP sequence represented by SEQ ID NO : 12, a linker chosen among SEQ ID NO : 16 to SEQ ID NO : 38, and the fragment delimited by the aminoacids located in positions 19 to 104 of the Cre recombinase, and represented by SEQ ID NO : 4, said first polypeptide sequence corresponding to SEQ ID NO : 44,
* the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FKBP sequence, the FKBP sequence represented by SEQ ID NO : 12, a linker chosen among SEQ ID NO : 16 to SEQ ID NO : 38, and the fragment delimited by the aminoacids located in positions 19 to 59 of the Cre recombinase, and represented by SEQ ID NO : 6, said first polypeptide sequence corresponding to SEQ ID NO : 46,
- and the second polypeptide sequence is chosen among the following sequences:
* the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FRB sequence, the FRB sequence represented by SEQ ID NO : 14, a linker chosen among SEQ ID NO : 16 to SEQ ID NO : 38, and the fragment delimited by the aminoacids located in positions 106 to 343 of the Cre recombinase, and represented by SEQ ID NO : 8, said first polypeptide sequence corresponding to SEQ ID NO : 48,
* the sequence comprising from the N-terminal extremity to the C-terminal extremity a Kozak sequence represented by SEQ ID NO : 42, a NLS signal represented by SEQ ID NO : 40, if necessary a linker of two aminoacids between the NLS signal and the FRB sequence, the FRB sequence represented by SEQ ID NO : 14, a linker chosen among SEQ ID NO : 16 to SEQ ID NO : 38, and the fragment delimited by the aminoacids located in positions 60 to 343 of the Cre recombinase, and represented by SEQ ID NO : 10, said first polypeptide sequence corresponding to SEQ ID NO : 50.

17. Nucleotide sequences encoding a polypeptide according to any of claims 9 to 16.

18. Nucleotide sequences according to claim 17 chosen among SEQ ID NO : 43, 45, 47, and 49, and coding respectively for the polypeptides sequences SEQ ID NO : 44, 46, 48, and 50.

19. Vectors, such as plasmids, containing a nucleotide sequence according to claim 17 or 18.

20. Cells transformed with a vector according to claim 19.

21. Process for regulating the activity of the P1 bacteriophage Cre recombinase as represented by SEQ ID NO : 2 in cells **characterized in that** it comprises:
- the transformation of cells with two vectors, one vector containing a first nucleotide sequence encoding a first polypeptide sequence as defined in claims 1 to 16, and the other vector containing a second sequence nucleotide encoding a second polypeptide sequence as defined in claims 1 to 16, or the transformation of cells with one vector containing said first and second nucleotide sequences,
- the addition to said cell of the third molecule as defined in claims 1 or 5, liable to form a complex with the first and second peptides comprised in the first and second polypeptide sequences mentioned above, thus leading to the physical association of the said first and second polypeptide sequences, and to a Cre recombinase activity.

22. Process for the *in vitro* inactivation of a selected gene **characterized in that** it comprises:
- the transformation of cells with two vectors, one vector containing a first sequence encoding a first polypeptide sequence as defined in claims 1 to 16, and the other vector containing a second sequence encoding a second polypeptide sequence as defined in claims 1 to 16, or the transformation of cells with one vector containing said first and second nucleotide sequences, said cells being also transformed in such way that said selected gene, or a functionally essential part of said gene, is flanked upstream and downstream with two sequences encoding LoxP, or equivalent sequences, such as Lox511, in parallel orientation,
- the addition to said transformed cells of the specific third molecule defined in claims 1 or 5, leading to a Cre recombinase activity and to the inactivation of said selected gene.

23. Process for inducing chromosomal deletion **characterized in that** it comprises:
- the transformation of cells with two vectors, one vector containing a first sequence encoding a first polypeptide sequence as defined in claims 1 to 16, and the other vector containing a second sequence encoding a second polypeptide sequence as defined in claims 1 to 16, or the transformation of cells with one vector containing said first and second nucleotide sequences, said cells being also transformed in such way that two sequences encoding LoxP, or equivalent sequences, in parallel orientation are introduced into a selected chromosome, delimitating the part to be excised from said chromosome,
- the addition to said transformed cells of the specific third molecule defined in claims 1 or 5, leading to a Cre recombinase activity and to the deletion of the LoxP-flanked chromosomal region.

24. Process for inducing reciprocal chromosomal translocation **characterized in that** it comprises:
- the transformation of cells with two vectors, one vector containing a first sequence encoding a first polypeptide sequence as defined in claims 1 to 16, and the other vector containing a second sequence encoding a second polypeptide sequence as defined in claims 1 to 16, or the transformation of cells with one vector containing said first and second nucleotide sequences, said cells being also transformed in such way that one sequence encoding LoxP, or equivalent sequence, is introduced into each of two selected chromosomes, at the sites at which the translocation should take place,
- the addition to said transformed cells of the specific third molecule defined in claims 1 or 5, leading to a Cre recombinase activity and to interchromosomal recombination.

25. Process for the *in vitro* activation of a selected gene **characterized in that** it comprises:
- the transformation of cells with two vectors, one vector containing a first sequence encoding a first polypeptide sequence as defined in claims 1 to 16, and the other vector containing a second sequence encoding a second polypeptide sequence as defined in claims 1 to 16, or the transformation of cells with one vector containing said first and second nucleotide sequences, said cells being also transformed in such way that a sequence containing a stop codon flanked with two sequences encoding LoxP, or equivalent sequences, of parallel orientation is inserted upstream said selected gene, in front of its start codon,
- the addition to said transformed cells of the specific third molecule defined in claims 1 or 5, leading to a Cre recombinase activity and to excision of said LoxP-flanked sequences and the transcription of said selected gene.

26. Process for exchanging one DNA cassette for another by recombinase-mediated cassette exchange **characterized in that** it comprises:
- the transformation of cells with two vectors, one vector containing a first sequence encoding a first polypeptide sequence as defined in claims 1 to 16, and the other vector containing a second sequence encoding a second polypeptide sequence as defined in claims 1 to 16 , or the transformation of cells with one vector containing said first and second nucleotide sequences, said cells being also transformed in such way that a target sequence is flanked by two sequences encoding LoxP, or equivalent sequences, containing complementary mutations within their palindromic arms, and one of them containing also a mutation within the spacer region,
- the introduction into said transformed cells of a targeting vector containing a cassette flanked by same variant of sequences encoding LoxP, or equivalent sequences,
- the addition to said transformed cells of the specific third molecule defined in claims 1 or 5, leading to a Cre recombinase activity and to the exchange of the target sequence with the cassette contained in the said targeting vector.

27. Process for the inactivation of a selected gene in transgenic animals **characterized in that** it comprises:
- the introduction into a transgenic line of two constructs, one construct containing a first sequence encoding a first polypeptide sequence as defined in claims 1 to 16, and the other construct containing a second sequence encoding a second polypeptide sequence as defined in claims 1 to 16, said transgenic line being homozygous for a modified allele of a selected gene that have been transformed in such way that said gene or a functionally essential part of said gene is flanked upstream and downstream with two sequences encoding LoxP, or equivalent sequences, in parallel orientation,
- the treatment of transgenic animals obtained from said transgenic line with the specific third molecule defined in claims 1 or 5, leading to a Cre recombinase activity and to inactivation of said selected gene.
